# EUROPEAN PATENT SPECIFICATION

(11) **EP 2 104 488 B1**
(45) Date of publication and mention of the grant of the patent: **12.10.2016**
(21) Application number: 07859130.2
(22) Date of filing: 19.12.2007
(51) Int. Cl.: A61K 39/00, A61K 47/00, A61K 9/127, A61K 49/22

(54) **GAS-FILLED MICROVESICLES COMPRISING THE FC-REGION OF AN ANTIBODY**
MIT GAS GEFÜLLTE MIKROVESIKEL ENTHALTEND DIE FC-REGION EINES ANTIKÖRPERS
MICROVÉSICULES REMPLIÉES AVEC GAZ CONTENANT LA RÉGION FC D'UN ANTICORPS

(30) Priority: 19.12.2006 US 641289
(43) Date of publication of application: 30.09.2009
(73) Proprietor: Bracco Suisse SA, 6928 Manno (CH)
(72) Inventor: ALLEMANN, Eric, 1228 Plan-les-Ouates (CH); BETTINGER, Thierry, 1228 Plan-les-ouates (CH); BUSSAT, Philippe, 1228 Plan-les-Ouates (CH)
(74) Representative: Ravizza, Claudio
(86) International application number: PCT/IB2007/004017
(87) International publication number: WO 2008/075191

(56) References cited:
- EP-A- 1 714 642
- WO-A-96/40285
- WO-A-99/13918
- WO-A-03/074566
- WO-A-2005/012493
- US-A1- 2005 070 693
- US-B1- 6 630 140

## Description

### Technical field

The present invention relates to gas filled microvesicles, particularly for use in ultrasound imaging, comprising a targeting compound or a therapeutic agent and a component having binding affinity for the Fc-region of said targeting compound or therapeutic agent. The invention further relates to a pharmaceutical kit comprising said gas-filled microvesicles or precursors thereof, and to a method for its preparation.

### Background of the invention

Rapid development of contrast agents in the recent years has generated a number of different formulations, which are useful in contrast-enhanced imaging of organs and tissue of human or animal body.

More recently, attention has been given to so-called "molecular imaging", where suitable target specific components are used in the formulation of the contrast agents, for allowing selective contrast-enhanced imaging of organs or tissues. In addition, therapeutic use of contrast agent formulations, optionally in combination with molecular imaging, has also been described.

A class of contrast agents, particularly useful for ultrasound contrast imaging, includes suspensions of gas bubbles of nano- and/or micro-metric size dispersed in an aqueous medium. Of particular interest are those formulations where the gas bubbles are stabilized, for example by using emulsifiers, oils, thickeners or sugars, or by entrapping or encapsulating the gas or a precursor thereof in a variety of systems. These stabilized gas bubbles are generally referred to in the art with various terminologies, such as, for instance, "microvesicles", "microspheres", "microbubbles", "microcapsules" or "microballoons". In the present description and claims, the term "gas-filled microvesicles" is used to identify any of the above described stabilized gas-bubbles.

The formulations of gas-filled microvesicles can be suitably modified, either for improving the diagnostic effect (e.g. through molecular imaging) and/or for therapeutic purposes, such as drug delivery and/or thrombolysis. For instance, gas-filled microvesicles can be associated (e.g. by inclusion in their boundary envelope) with therapeutic agents and/or with specific components which are capable to link to a determined target within a patient's body (known as "targeting ligands"). Examples of targeting ligands include, for instance, peptides, proteins or antibodies, capable of binding to specific organ or tissue such as, for instance, angiogenic tissue or blood clots.

A possible way to associate a targeting ligand or a therapeutic compound to the structure of a microvesicle is to bind it to suitable molecules which can be employed for the formation of the microvesicles envelope. The targeting or therapeutic component can be directly linked to the envelope-forming molecule or through a suitable spacer, in general a polymeric spacer. The binding can be either covalent or non-covalent, e.g. through an affinity binding pair.

Association of targeting ligands or therapeutic agents to microvesicles through a spacer is disclosed, for instance, in WO 96/40285, WO 98/18501, WO 98/53857.

WO03074566, WO9913918, EP714642, WO2005012493, US630140, US2005070693 reveal varied antibody-containing preparations.

In general, known methods for binding a targeting ligand or therapeutic compound to a microvesicle require an interaction between respective moiety pairs (e.g., either through covalent binding or non-covalent binding pairs) which are specific, i.e. each moiety of the pair specifically reacts with or binds to the other respective moiety of the pair.

The Applicant has now found a new assembly of functionalized gas-filled microvesicles, where the targeting ligand or therapeutic compound is a component associated to the microvesicle by means of a non covalent interaction between the Fc-region of said component and a second component capable of associating with said Fc-region of the antibody.

### Summary of the invention

An aspect of the invention relates to a gas-filled microvesicle, said microvesicle comprising a boundary envelope containing said gas, wherein said microvesicle comprises:
- a first component, bound to said envelope, having binding affinity for a Fc-region of an antibody; and
- a second component comprising a Fc-region of an antibody, bound to said first component through said Fc-region, said second component comprising a targeting ligand or a therapeutic agent.

According to a preferred embodiment of the invention, the second component is an antibody or a chimeric protein.

According to a preferred embodiment, said first component is a protein, preferably selected from the group comprising protein G, protein A and recombinant protein A/G protein, or an anti-Fc antibody.

A further aspect of the invention relates to a suspension of gas-filled microvesicles as above defined in a physiologically acceptable aqueous carrier.

A further aspect of the invention relates to a kit comprising:
- a gas-filled microvesicle, or precursor thereof, comprising a first component having binding affinity for a Fc-region of an antibody; and
- a second component, comprising a Fc-region capable of binding to said first component through said Fc-region.

### Figures

Figure 1 is a schematic representation of an antibody.
Figure 2 is a schematic representation of a chimeric protein.
Figure 3 is a schematic representation of a microvesicle of the invention binding to a biological target.

### Detailed description of the invention

The term "protein" includes proteins of natural or recombinant origin. Examples of proteins of natural origin are, for instance, those originating from the wall of bacteria, such as protein G (from streptococcal strains) or protein A from (staphylococcal strains). Recombinant proteins can be obtained, for instance through genetic engineering (also called gene splicing or recombinant DNA technology), by inserting a foreign gene (responsible for encoding a desired protein) into the genetic material of bacteria or yeast cells, to produce the desired protein. Recombinant proteins include also "fusion protein", i.e. protein being the result of the translation of two or more genes joined such that they retain their correct reading frames but make a single protein.

The term "Fc-binding component" inlcudes any component having substantial binding affinity for the Fc-region of an antibody. In particular, said component is capable of stably non-covalently binding the Fc-region of an antibody, at physiological pH values (typically from 5 to 8). Fc-binding components include proteins and fragments thereof, anti-Fc antibodies and fragments thereof, peptide sequences and receptors.

The term "anti-Fc antibody" includes antibodies recognizing and having a particular affinity for the Fc-region of another antibody.

The term "Fc-receptor", refers to a receptor of a Fc-region of an antibody and includes in particular purified receptors and recombinant receptors obtained from those naturally found at the surface of cells of the immune system (such as macrophages, neutrophils, eosinophils) capable of binding to a Fc-region of an antibody.

The term "fragment", in particular when referred to a fragment of a protein or anti-Fc antibody having binding affinity for the Fc-region of antibodies, includes proteins or peptidic sequences, which are partial sequences of an entire protein or antibody and that also bind to Fc fragments of antibodies.

The expression "Fc-region of an antibody" identifies the "Fragment crystallisable region" of an antibody (also known in the art as the "constant domain" of the antibody), which is generally formed by the interaction between the two heavy peptide chains of the antibody. The expression includes either Fc-region normally found in antibodies structures, as well as Fc-region of chimeric fusion proteins prepared from a gene coding for a Fc-region of an antibody.

The expression "Fc-comprising component" includes any compound comprising a Fc-region of an antibody in its structure, such as antibodies or chimeric proteins.

The expression "chimeric protein containing a Fc-region of an antibody" includes recombinant fusion proteins created through the joining of a first gene, which originally codes for a Fc-region of an antibody, and of at least a second gene, coding for at least one protein of interest (e.g. for targeting or therapeutic purposes). Translation of this fusion gene results in a single protein with function properties derived from each of the proteins originally encoded by the respective genes, namely the function of the Fc-region and the function of the other protein.

"Non-covalent binding" includes intermolecular interactions among two or more molecules which do not involve a covalent bond such as, for instance, ionic or electrostatic interactions, dipole-dipole interactions, hydrogen bonding, hydrophilic or hydrophobic interactions, van der Waal's forces and combinations thereof.

The term "gas-filled microvesicles" includes any structure comprising bubbles of gas of micrometric or nanometric size surrounded by an envelope or layer (including film-forming layers) of a stabilizing material. The term includes what is known in the art as gas-filled liposomes, microbubbles, microspheres, microballoons or microcapsules. The stabilizing material can be any material typically known in the art including, for instance, surfactants, lipids, sphingolipids, oligolipids, phospholipids, proteins, polypeptides, carbohydrates, and synthetic or natural polymeric materials.

The term "microbubbles" includes aqueous suspensions in which the bubbles of gas are bounded at the gas/liquid interface by a very thin envelope (film) involving a stabilizing amphiphilic material disposed at the gas to liquid interface (sometimes referred to in the art as an "evanescent" envelope). Microbubble suspensions can be prepared by contacting a suitable precursor thereof, such as powdered amphiphilic materials (e.g. freeze-dried preformed liposomes or freeze-dried or spray-dried phospholipid dispersions or solutions) with air or other gas and then with an aqueous carrier, while agitating to generate a microbubble suspension which can then be administered, preferably shortly after its preparation. Examples of aqueous suspensions of gas microbubbles, of precursors and of the preparation thereof are disclosed, for instance, in US 5,271,928, US 5,445,813, US 5,413,774, US 5,556,610, 5,597,549, US 5,827,504 and WO 04/069284, which are here incorporated by reference in their entirety.

The terms "microballoons" or "microcapsules" include suspensions in which the bubbles of gas are surrounded by a solid material envelope of a lipid or of natural or synthetic polymers. Examples of microballoons and of the preparation thereof are disclosed, for instance, in US 5,711,933 and US 6,333,021.

The term "targeting ligand" includes any compound, moiety or residue having, or being capable of promoting a targeting activity towards tissues and/or receptors *in vivo.* Targets with which a targeting ligand may be associated include tissues such as, for instance, myocardial tissue (including myocardial cells and cardiomyocytes), membranous tissues (including endothelium and epithelium), laminae, connective tissue (including interstitial tissue) or tumors; blood clots; and receptors such as, for instance, cell-surface receptors for peptide hormones, neurotransmitters, antigens, complement fragments and immunoglobulins.

The term "targeted gas-filled microvesicle" includes any gas-filled microvesicle comprising at least one targeting ligand in the form of a Fc-comprising component in its formulation.

The phrase "intermediate of a targeted gas-filled microvesicle" includes any gas-filled microvesicle which can be converted into a targeted gas-filled microvesicle. Such intermediate may include, for instance, gas-filled microvesicles (or precursors thereof) including a suitable reactive moiety (e.g. maleimide), which can be reacted with a corresponding complementary reactive (e.g. thiol) linked to a targeting ligand.

The term "therapeutic agent" includes within its meaning any compound, moiety or residue which may be used in any therapeutic application, such as in methods for the treatment of a disease in a patient, as well as any substance which is capable of exerting or responsible to exert a biological effect in vitro and/or in vivo. Therapeutic agents thus include any compound or material capable of being used in the treatment (including prevention, alleviation, pain relief or cure) of any pathological status in a patient (including malady, affliction, disease, lesion or injury). Examples of therapeutic agents are drugs, pharmaceuticals, bioactive agents, cytotoxic agents, chemotherapy agents, radiotherapeutic agents, proteins, natural or synthetic peptides, including oligopeptides and polypeptides, vitamins, steroids and genetic material, including nucleosides, nucleotides, oligonucleotides, polynucleotides and plasmids.

The expression "physiologically acceptable aqueous carrier" includes liquid carriers which are generally employed for injections in animals, such as, for instance, water, typically sterile, pyrogen free water (to prevent as much as possible contamination in the intermediate lyophilized product), aqueous solutions such as saline (which may advantageously be balanced so that the final product for injection is not hypotonic), or aqueous solutions of one or more tonicity adjusting substances such as salts or sugars, sugar alcohols, glycols or other non-ionic polyol materials (eg. glucose, sucrose, sorbitol, mannitol, glycerol, polyethylene glycols, propylene glycols and the like).

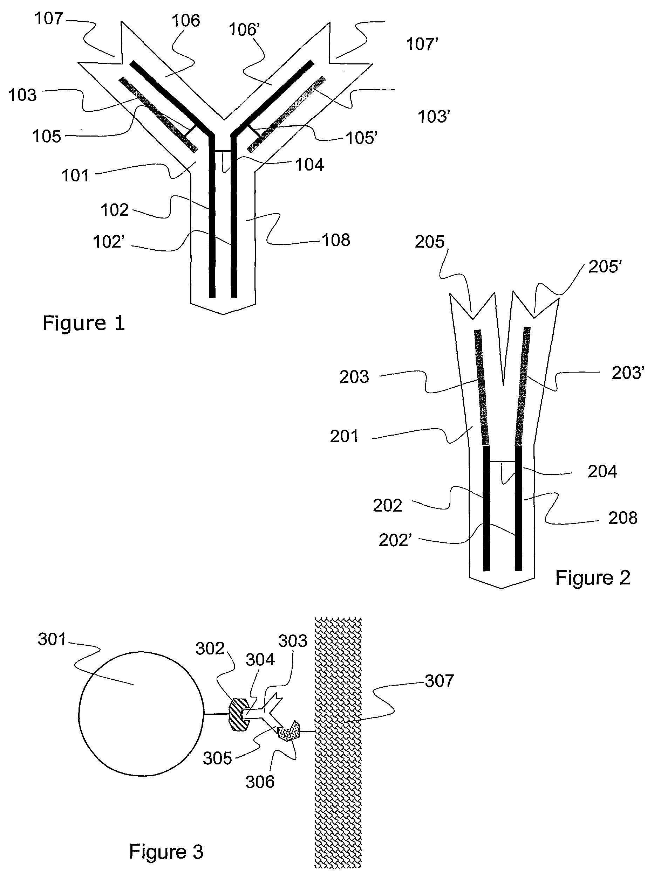

As know in the art, antibodies (also known as "immunoglobulins") are glycoproteins which may schematically be represented as Y-shaped molecules, as illustrated in figure 1. The antibody (101) includes two heavy polypeptide chains (102, 102') and two light polypeptide chains (103, 103'). One or more disulfide bonds are present between the heavy chains (104) and between the respective light and heavy chains (105, 105'). The arms (106, 106') of the Y-shaped molecule are known in the art as the Fab region of the antibody ("Fragment antigen binding region"); each arm contains a site (107, 107') capable of binding to an antigen or receptor. The Fab region of antibodies is also identified as the "variable" domain of the antibody, responsible for the recognition of a variety of antigens or receptors. The base (108) of the Y-shaped structure of the antibody is identified in the art as the Fc-region of the antibody and plays a role in the immune regulation function of the antibody. The Fc-region of antibodies is also identified as the "constant portion" of the antibody, as the peptide sequences of the heavy chains in the Fc-region of the different antibodies have certain similarities, in particular among antibodies belonging to a same class (IgG, IgA, IgM, IgE and IgD) and more particularly in those belonging to a same subclass (e.g. IgG1, IgG2, IgG3, IgG4 and IgA1, IgA2). The substantial similarity of the Fc-region among different antibodies allows a component (e.g. protein, fragment, peptide sequence or receptor) having binding affinity for said Fc-region to bind said antibodies. In particular, some binding components (e.g. anti-Fc antibodies) may have affinity for the Fc-region of a single class (or subclass) of antibodies, while some other components (such as proteins, in particular proteins G, A or A/G) are advantageously able to bind Fc-regions of different classes or subclasses of antibodies.

Figure 2 schematically illustrates the structure of a chimeric protein (201). The chimeric protein comprises two residues (202, 202') of heavy peptide chains of an antibody, linked by at least one disulfide bond (204), which form the Fc portion of the chimeric protein. Two peptide chains (203, 203') are bound to respective residues (202, 202') and define respective sites (205, 205') capable of binding to an antigen or receptor. Depending on the specific protein, the binding site can be located at different positions along the structure.

Thanks to the fact that the component comprising a Fc-region (e.g. an antibody or chimeric protein as illustrated above) binds to the microvesicle through its Fc-region, the microbubbles of the invention have the advantage that the component is presented to the biological receptor in the correct orientation, i.e. with the targeting ligand or therapeutic agent positioned outwards with respect to the microvesicles envelope and ready to bind to a specific receptor. Figure 3 illustrates the above advantage with respect to a targeting antibody bound through its Fc-region to a microvesicle. The schematic drawing of figure 2 illustrates a gas-filled microvesicle (301), which bears a Fc-binding component (302), e.g. protein G, to which an antibody (303) is bound through its Fc-region (304). For the sake of clarity, only one Fc-binding component, with the respective bound antibody, is represented. The Fab region (305) of the antibody allows in turn the binding of the microvesicle construct on the biological target receptor (306) expressed on the tissue (307) under investigation.

A further advantage of the microvesicles of the invention is that their preparation does not involve any chemical modification of the Fc-comprising component to be bound to the molecule, as the binding of said component to the microvesicles containing the Fc-binding component is effected through the Fc-region already present in the component.

In addition, the linking of the Fc-comprising component through its Fc-region allows the preparation of a single intermediate microvesicle constructs, containing the suitable Fc-binding component, which may thus be used to bind a plurality of different Fc-comprising component capable of binding to said Fc-binding component.

According to an embodiment of the present invention, the gas-filled microvesicles are microbubbles.

Amphiphilic components suitable for forming a stabilizing envelope of microbubbles comprise, for instance, phospholipids; lysophospholipids; fatty acids, such as palmitic acid, stearic acid, arachidonic acid or oleic acid; lipids bearing polymers, such as chitin, hyaluronic acid, polyvinylpyrrolidone or polyethylene glycol (PEG), also referred as "pegylated lipids"; lipids bearing sulfonated mono- di-, oligo- or polysaccharides; cholesterol, cholesterol sulfate or cholesterol hemisuccinate; tocopherol hemisuccinate; lipids with ether or ester-linked fatty acids; polymerized lipids; diacetyl phosphate; dicetyl phosphate; ceramides; polyoxyethylene fatty acid esters (such as polyoxyethylene fatty acid stearates), polyoxyethylene fatty alcohols, polyoxyethylene fatty alcohol ethers, polyoxyethylated sorbitan fatty acid esters, glycerol polyethylene glycol ricinoleate, ethoxylated soybean sterols, ethoxylated castor oil or ethylene oxide (EO) and propylene oxide (PO) block copolymers; sterol aliphatic acid esters including, cholesterol butyrate, cholesterol iso-butyrate, cholesterol palmitate, cholesterol stearate, lanosterol acetate, ergosterol palmitate, or phytosterol n-butyrate; sterol esters of sugar acids including cholesterol glucuronides, lanosterol glucoronides, 7-dehydrocholesterol glucoronide, ergosterol glucoronide, cholesterol gluconate, lanosterol gluconate, or ergosterol gluconate; esters of sugar acids and alcohols including lauryl glucoronide, stearoyl glucoronide, myristoyl glucoronide, lauryl gluconate, myristoyl gluconate, or stearoyl gluconate; esters of sugars with aliphatic acids including sucrose laurate, fructose laurate, sucrose palmitate, sucrose stearate, glucuronic acid, gluconic acid or polyuronic acid; saponins including sarsasapogenin, smilagenin, hederagenin, oleanolic acid, or digitoxigenin; glycerol or glycerol esters including glycerol tripalmitate, glycerol distearate, glycerol tristearate, glycerol dimyristate, glycerol trimyristate, glycerol dilaurate, glycerol trilaurate, glycerol dipalmitate; long chain alcohols including n-decyl alcohol, lauryl alcohol, myristyl alcohol, cetyl alcohol, or n-octadecyl alcohol; 6-(5-choiesten-3β-yloxy)-1-thio-β-D-galactopyranoside; digalactosyldiglyceride; 6-(5-cholesten-3 β -yloxy)hexyl-6-amino-6-deoxy-1-thio- β -D-galactopyranoside; 6-(5-cholesten-3 β -yloxy)hexyl-6-amino-6-deoxyl-1-thio-β-D-mannopyranoside; 12-(((7'-diethylaminocoumarin-3-yl)carbonyl)methylamino)octadecanoic acid; N-[12-(((7'-diethylaminocoumarin-3-yl)carbonyl)methylamino)octadecanoyl]-2-aminopalmitic acid; N-succinyl-dioleylphosphatidylethanolamine; 1,2-dioleyl-*sn*-glycerol; 1,2-dipalmitoyl-*sn*-3-succinylglycerol; 1,3-dipalmitoyl-2-succinylglycerol; 1-hexadecyl-2-palmitoylglycerophosphoethanolamine or palmitoylhomocysteine; alkylamines or alkylammonium salts, comprising at least one (C₁₀-C₂₀), preferably (C₁₄-C₁₈), alkyl chain, such as, for instance, N-stearylamine, N,N'-distearylamine, N-hexadecylamine, N,N'-dihexadecylamine, N-stearylammonium chloride, N,N'-distearylammonium chloride, N-hexadecylammonium chloride, N,N'-dihexadecylammonium chloride, dimethyldioctadecylammonium bromide (DDAB), hexadecyltrimethylammonium bromide (CTAB); tertiary or quaternary ammonium salts comprising one or preferably two (C₁₀-C₂₀), preferably (C₁₄-C₁₈), acyl chain linked to the N-atom through a (C₃-C₆) alkylene bridge, such as, for instance, 1,2-distearoyl-3-trimethylammonium-propane (DSTAP), 1,2-dipalmitoyl-3-trimethylammonium-propane (DPTAP), 1,2-oleoyl-3-trimethylammonium-propane (DOTAP), 1,2-distearoyl-3-dimethylammonium-propane (DSDAP); and mixtures or combinations thereof.

Depending on the combination of components and on the manufacturing process of the microbubbles, the above listed exemplary compounds may be employed as the main compound for forming the microbubble's envelope or as simple additives, thus being present only in minor amounts.

According to a preferred embodiment, at least one of the compounds forming the microbubbles' envelope is a phospholipid, optionally in admixture with any of the other above-cited materials. According to the present description, the term phospholipid is intended to encompass any amphiphilic phospholipid compound, the molecules of which are capable of forming a stabilizing film of material (typically in the form of a mono-molecular layer) at the gas-water boundary interface in the final microbubbles suspension. Accordingly, these materials are also referred to in the art as "film-forming phospholipids".

Amphiphilic phospholipid compounds typically contain at least one phosphate group and at least one, preferably two, lipophilic long-chain hydrocarbon groups.

Examples of suitable phospholipids include esters of glycerol with one or preferably two (equal or different) residues of fatty acids and with phosphoric acid, wherein the phosphoric acid residue is in turn bound to a hydrophilic group, such as, for instance, choline (phosphatidylcholines - PC), serine (phosphatidylserines - PS), glycerol (phosphatidylglycerols - PG), ethanolamine (phosphatidylethanolamines - PE), inositol (phosphatidylinositol). Esters of phospholipids with only one residue of fatty acid are generally referred to in the art as the "lyso" forms of the phospholipid or "lysophospholipids". Fatty acids residues present in the phospholipids are in general long chain aliphatic acids, typically containing from 12 to 24 carbon atoms, preferably from 14 to 22; the aliphatic chain may contain one or more unsaturations or is preferably completely saturated. Examples of suitable fatty acids included in the phospholipids are, for instance, lauric acid, myristic acid, palmitic acid, stearic acid, arachidic acid, behenic acid, oleic acid, linoleic acid, and linolenic acid. Preferably, saturated fatty acids such as myristic acid, palmitic acid, stearic acid and arachidic acid are employed.

Further examples of phospholipids are phosphatidic acids, i.e. the diesters of glycerol-phosphoric acid with fatty acids; sphingolipids such as sphingomyelins, i.e. those phosphatidylcholine analogs where the residue of glycerol diester with fatty acids is replaced by a ceramide chain; cardiolipins, i.e. the esters of 1,3-diphosphatidylglycerol with a fatty acid; glycolipids such as gangliosides GM1 (or GM2) or cerebrosides; glucolipids; sulfatides and glycosphingolipids.

As used herein, the term phospholipids include either naturally occurring, semisynthetic or synthetically prepared products that can be employed either singularly or as mixtures.

Examples of naturally occurring phospholipids are natural lecithins (phosphatidylcholine (PC) derivatives) such as, typically, soya bean or egg yolk lecithins.

Examples of semisynthetic phospholipids are the partially or fully hydrogenated derivatives of the naturally occurring lecithins. Preferred phospholipids are fatty acid di-esters of phosphatidylcholine, ethylphosphatidylcholine, phosphatidylglycerol, phosphatidic acid, phosphatidylethanolamine, phosphatidylserine, phosphatidylinositol or of sphingomyelin.

Examples of preferred phospholipids are, for instance, dilauroyl-phosphatidylcholine (DLPC), dimyristoyl-phosphatidylcholine (DMPC), dipalmitoyl-phosphatidylcholine (DPPC), diarachidoyl-phosphatidylcholine (DAPC), distearoyl-phosphatidylcholine (DSPC), dioleoyl-phosphatidylcholine (DOPC), 1,2 Distearoyl-*sn*-glycero-3-Ethylphosphocholine (Ethyl-DSPC), dipentadecanoyl-phosphatidylcholine (DPDPC), 1-myristoyl-2-palmitoyl-phosphatidylcholine (MPPC), 1-palmitoyl-2-myristoyl-phosphatidylcholine (PMPC), 1-palmitoyl-2-stearoyl-phosphatidylcholine (PSPC), 1-stearoyl-2-palmitoyl-phosphatidylcholine (SPPC), 1-palmitoyl-2-oleylphosphatidylcholine (POPC), 1-oleyl-2-palmitoyl-phosphatidylcholine (OPPC), dilauroyl-phosphatidylglycerol (DLPG) and its alkali metal salts, diarachidoylphosphatidyl-glycerol (DAPG) and its alkali metal salts, dimyristoylphosphatidylglycerol (DMPG) and its alkali metal salts, dipalmitoylphosphatidylglycerol (DPPG) and its alkali metal salts, distearoylphosphatidylglycerol (DSPG) and its alkali metal salts, dioleoyl-phosphatidylglycerol (DOPG) and its alkali metal salts, dimyristoyl phosphatidic acid (DMPA) and its alkali metal salts, dipalmitoyl phosphatidic acid (DPPA) and its alkali metal salts, distearoyl phosphatidic acid (DSPA), diarachidoylphosphatidic acid (DAPA) and its alkali metal salts, dimyristoyl-phosphatidylethanolamine (DMPE), dipalmitoylphosphatidylethanolamine (DPPE), distearoyl phosphatidyl-ethanolamine (DSPE), dioleylphosphatidyl-ethanolamine (DOPE), diarachidoylphosphatidylethanolamine (DAPE), dilinoleylphosphatidylethanolamine (DLPE), dimyristoyl phosphatidylserine (DMPS), diarachidoyl phosphatidylserine (DAPS), dipalmitoyl phosphatidylserine (DPPS), distearoylphosphatidylserine (DSPS), dioleoylphosphatidylserine (DOPS), dipalmitoyl sphingomyelin (DPSP), and distearoylsphingomyelin (DSSP), dilauroyl-phosphatidylinositol (DLPI), diarachidoylphosphatidylinositol (DAPI), dimyristoylphosphatidylinositol (DMPI), dipalmitoylphosphatidylinositol (DPPI), distearoylphosphatidylinositol (DSPI), dioleoyl-phosphatidylinositol (DOPI).

Suitable phospholipids further include phospholipids modified by linking a hydrophilic polymer, such as polyethyleneglycol (PEG) or polypropyleneglycol (PPG), thereto. Preferred polymer-modified phospholipids include "pegylated phospholipids", i.e. phospholipids bound to a PEG polymer. Examples of pegylated phospholipids are pegylated phosphatidylethanolamines ("PE-PEGs" in brief) i.e. phosphatidylethanolamines where the hydrophilic ethanolamine moiety is linked to a PEG molecule of variable molecular weight (e.g. from 300 to 5000 daltons), such as DPPE-PEG (or DSPE-PEG, DMPE-PEG or DAPE-PEG). For example, DPPE-PEG2000 refers to DPPE having attached thereto a PEG polymer having a mean average molecular weight of about 2000.

Particularly preferred phospholipids are DAPC, DSPC, DSPG, DPPA, DSPA, DMPS, DPPS, DSPS and Ethyl-DSPC. Most preferred are DSPG or DSPC.

Mixtures of phospholipids can also be used, such as, for instance, mixtures of DSPE, DPPE, DPPC, DSPC and/or DAPC with DSPS, DPPS, DSPA, DPPA, DSPG, DPPG, Ethyl-DSPC and/or Ethyl-DPPC.

In preferred embodiments, the phospholipid is the main component of the stabilizing envelope of microbubbles, amounting to at least 50% (w/w) of the total amount of components forming the envelope of the gas-filled microbubbles. In some of the preferred embodiments, substantially the totality of the envelope (i.e. at least 80% and up to 100% by weight) can be formed of phospholipids.

The phospholipids can conveniently be used in admixture with any of the above listed amphiphilic compounds. Thus, for instance, substances such as cholesterol, ergosterol, phytosterol, sitosterol, lanosterol, tocopherol, propyl gallate or ascorbyl palmitate, fatty acids such as myristic acid, palmitic acid, stearic acid, arachidic acid and derivatives thereof or butylated hydroxytoluene and/or other non-phospholipid compounds can optionally be added to one or more of the foregoing phospholipids in proportions ranging from zero to 50% by weight, preferably up to 25%. Particularly preferred is palmitic acid.

According to a preferred embodiment, the envelope of microbubbles according to the invention includes a compound bearing an overall (positive or negative) net charge. Said compound can be a charged amphiphilic material, preferably a lipid or a phospholipid.

Examples of phospholipids bearing an overall negative charge are derivatives, in particular fatty acid di-ester derivatives, of phosphatidylserine, such as DMPS, DPPS, DSPS; of phosphatidic acid, such as DMPA, DPPA, DSPA; of phosphatidylglycerol such as DMPG, DPPG and DSPG or of phosphatidylinositol, such as DMPI, DPPI or DPPI. Also modified phospholipids, in particular PEG-modified phosphatidylethanolamines, such as DPPE-PEG or DSPE-PEG, can be used as negatively charged molecules. Also the lyso- form of the above cited phospholipids, such as lysophosphatidylserine derivatives (e.g. lyso-DMPS, -DPPS or -DSPS), lysophosphatidic acid derivatives (e.g. lyso-DMPA, -DPPA or -DSPA) and lysophosphatidylglycerol derivatives (e.g. lyso-DMPG, - DPPG or -DSPG), can advantageously be used as negatively charged compounds. Other examples of negatively charged compounds are bile acid salts such as cholic acid salts, deoxycholic acid salts or glycocholic acid salts; and (C₁₂-C₂₄),preferably (C₁₄-C₂₂) fatty acid salts such as, for instance, palmitic acid salts, stearic acid salts, 1,2-dipalmitoyl-*sn*-3-succinylglycerol salts or 1,3-dipalmitoyl-2-succinylglycerol salts.

Preferably, the negatively charged compound is selected among DPPA, DPPS, DSPG, DPPG, DSPE-PEG2000, DSPE-PEG5000 or mixtures thereof.

The negatively charged component is typically associated with a corresponding positive counter-ion, which can be mono- (e.g. an alkali metal or ammonium), di- (e.g. an alkaline earth metal) or tri-valent (e.g. aluminium). Preferably the counter-ion is selected among alkali metal cations, such as Li⁺, Na⁺, or K⁺, more preferably Na⁺.

Examples of phospholipids bearing an overall positive charge are derivatives of ethylphosphatidylcholine, in particular di-esters of ethylphosphatidylcholine with fatty acids, such as 1,2-distearoyl-*sn*-glycero-3-ethylphosphocholine (Ethyl-DSPC or DSEPC), 1,2-dipalmitoyl-*sn*-glycero-3-ethylphosphocholine (Ethyl-DPPC or DPEPC). The negative counterion is preferably a halide ion, in particular chloride or bromide ion. Examples of positively charged compounds that can be incorporated into the envelope of microbubbles are mono-, di- tri-, or tetra-alkylammonium salts with a halide counter ion (e.g. chloride or bromide) comprising at least one (C₁₀-C₂₀), preferably (C₁₄-C₁₈), alkyl chain, such as, for instance mono- or di-stearylammonium chloride, mono or di-hexadecylammonium chloride, dimethyldioctadecylammonium bromide (DDAB) or hexadecyltrimethylammonium bromide (CTAB). Further examples of positively charged compounds that can be incorporated into the envelope of microbubbles are tertiary or quaternary ammonium salts with a halide counter ion (e.g. chloride or bromide) comprising one or preferably two (C₁₀-C₂₀), preferably (C₁₄-C₁₈), acyl chains linked to the N-atom through a (C₃-C₆) alkylene bridge, such as, for instance, 1,2-distearoyl-3-trimethylammonium-propane (DSTAP), 1,2-dipalmitoyl-3-trimethylammonium-propane (DPTAP), 1,2-oleoyl-3-trimethylammonium-propane (DOTAP) or 1,2-distearoyl-3-dimethylammonium-propane (DSDAP).

DSEPC, DPEPC and/or DSTAP are preferably employed as positively charged compounds in the microbubble envelope.

The positively charged component is typically associated with a corresponding negative counter-ion, which can be mono- (e.g. halide), di- (e.g. sulphate) or tri-valent (e.g. phosphate). Preferably the counter-ion is selected from among the halide ions, such as F⁻ (fluorine), Cl⁻ (chlorine) or Br⁻ (bromine).

Mixtures of neutral and charged compounds, in particular of phospholipids and/or lipids, can be satisfactorily employed to form the microbubble envelope. The amount of charged lipid or phospholipid may vary from about 95 mol % to about 1 mol %, with respect to the total amount of lipid and phospholipid, preferably from 80 mol % to 2.5 mol %.

Preferred mixtures of neutral phospholipids and charged lipids or phospholipids are, for instance, DPPG/DSPC, DSTAP/DAPC, DPPS/DSPC, DPPS/DAPC, DPPE/DPPG, DSPA/DAPC, DSPA/DSPC and DSPG/DSPC.

In addition to the above amphiphilic components suitable for forming a stabilizing envelope of microbubbles, microbubbles according to the invention further comprise a Fc-binding component.

Examples of suitable Fc-binding components include proteins having binding affinity for the Fc-region of antibodies such as, for instance, natural or recombinant protein G or A protein or recombinant fusion protein A/G.

Recombinant protein G is commercially available, for instance, from BioVision (Mountain View, CA, USA). The recombinant protein G (tailored to maximize specific binding to Fc-regions) corresponds to the amino acid sequence 190-384 of the Streptococcus sp. protein G Ig binding domains with 6x His-tag on N-terminus; molecular weight of 26.1 kDa; Gene Bank Accession Number CAA27638.

Recombinant protein A is also commercially available, for instance, from BioVision (Mountain View, CA, USA). The recombinant protein A (tailored to maximize specific binding to Fc-regions) corresponds to the amino acid sequence 32-327 of the Staphylococcus aureus subsp. aureus protein A Ig binding domains with 6x His-tag on N-terminus; molecular weight of 38.9 kDa; Gene Bank Accession Number YP_498670. Cell wall binding region, albumin binding region and other non-specific binding regions have been eliminated from the recombinant protein to ensure the maximum specific binding to Fc-regions.

Recombinant fusion protein A/G is also available for instance, from BioVision (Mountain View, CA, USA). It is a genetically engineered protein that combines the IgG binding profiles of both protein A and protein G. Recombinant fusion protein A/G contains 6x His-tag on the N-terminus, five Ig-binding regions of protein A fusion with three Ig-binding region of protein G. Cell wall binding region, albumin binding region and other non-specific binding regions have been eliminated from the fusion protein A/G to ensure the maximum specific IgG binding. 6x His-tag on N-terminus can be used for affinity purification or for protein A/G detection using anti-His-tag antibody. Protein A/G binds to all IgG subclasses from various mammalian species.

Additional Fc-binding components include fragments of said proteins such as, for instance, a fragment of the B1 domain of protein G, which corresponds to GB1 Hairpin Peptide (Streptococcus sp.); IgG binding protein G (amino acid sequence 267 to 282 of native Streptococcus sp.) protein G B1 domain, (or amino acid sequence 41 to 56 of B1 domain (CAS Number 160291-75-8), is available from Bachem A.G. (Bubbendorf, Switzerland).

Further Fc-binding components which may advantageously be employed include anti-Fc antibodies. Anti-Fc antibodies are generally identified according to the following convention:
"(animal species A) anti-(animal species B) Ig(X) Fc",
where the expression "animal species A" identifies the animal species in which the antibody is produced; the expression "animal species B" identifies the animal species in which the anti-Fc antibody recognizes the Fc-region of the selected immunoglobulin Ig; and Ig(X) identifies the class (and, where applicable, also the subclass) of immunoglobulins (i.e. IgG, IgA, IgE, etc.), the Fc-region of which is recognized by the specific anti-Fc antibody.

Thus, for instance, a "goat anti-rat IgG Fc" is an antibody produced in a goat reacting specifically with the Fc-region of class G immunoglobulins of rats.

"Animal species A" can be, for instance, rabbit, rat, mouse, goat or monkey, and "Animal species B" can be any species different from species A such as, selected for instance among human, mouse, rat, rabbit, or goat.

In general, if the Fc-binding component is an "(animal species A) anti-(animal species B) IgG Fc" antibody, such Fc-binding component will bind at least all the IgG of the animal species B; not rarely, the same antibody may bind also different classes of immunoglobulins as well as immunoglobulins of different animal species. As the binding of the Fc-region is effected through the Fab-regions of the anti-Fc antibody, it is apparent that, unless sterically prevented, each anti-Fc antibody is capable of binding two antibodies through their respective Fc-regions.

Examples of commercially available anti-Fc antibodies are, for instance, "goat anti-rat IgG Fc" (Chemicon - Millipore group, Billerica, MA, USA); "mouse anti-human Fc" (Serotec, Raleigh, NC, USA), "goat anti-guinea pig Fc" (Serotec, Raleigh, NC, USA), Anti-Rat IgG (Fc) (Beckman Coulter, Fullerton,CA, U.S.A).

Furthermore, also Fc-receptors may be used as Fc-binding components, such as, for instance, the Fc-gamma receptors (FcγR), that bind the most common class of antibody, IgG; , the Fc-alpha receptors (FcαR), that bind antibody of the IgA class; and the Fc-epsilon receptors (FcεR), that bind antibody of the IgE class. Fcγr receptors include several members such as, for instance, FcγRI (CD64), FcγRIIA (CD32), FcγRIIB (CD32), FcγRIIIA (CD16a), FcγRIIIB (CD16b), differing in their antibody affinities and in their molecular structure. For instance, FcγRI binds to IgG more strongly than FcγRII and FcγRIII. FcγRI is available, for instance, as a recombinant protein from Abnova Corporation (Taipei City, Taiwan).

The Fc-binding component can be associated to or incorporated in the stabilizing envelope of the microbubble according to conventional methods, for instance by covalently binding the Fc-binding component to an amphiphilic component forming the stabilizing envelope of the microbubble (in brief "envelope-forming component"). Said component can be selected among those previously illustrated, particularly preferred being phospholipids, in particular phosphatidylethanolamines (e.g. DSPE or DPPE). The Fc-binding component can be linked directly to the envelope-forming component, e.g. by means of a covalent bond involving reactive groups contained in the respective components, thus obtaining a Fc-binding/envelope-forming construct. Alternatively, a spacer component can be introduced between the Fc-binding component and the envelope-forming component, to obtain a Fc-binding/spacer/envelope-forming construct. Examples of suitable spacers include, for instance, hydrophilic synthetic polymers such as, polyethylenglycol, polyvinylpyrrolidone, polyacrylic acid, polyhydroxymethyl acrylate. Preferably, polyethylenglycol (PEG) is employed. The synthetic polymer may include from 2 to about 500 monomer units, preferably from about 12 to about 250 and even more preferably from about 20 to about 130 monomer units.

The reacting components may either contain the desired reactive groups or can be modified ("functionalized") according to conventional techniques to include the desired reactive group into the component.

For instance, if one of the two reacting components includes a reactive amino group, it can be reacted with the other component containing a suitable corresponding reactive moiety, such as an isothiocyanate group (to form a thiourea bond), a reactive ester (to form an amide bond), or an aldehyde group (to form an imine bond, which may be reduced to an alkylamine bond). Alternatively, if one of the two reacting components includes a reactive thiol group, suitable complementary reactive moieties on the other component may include haloacetyl derivatives, maleimides (to form a thioether bond) or a mixed disulfide comprising a sulphide in the form of a 2-pyridylthio group which upon reaction with a thiol derived from the thiol-bearing component results in the formation of a stable disulfide bond between the two components. Furthermore, if a one of the two reacting components includes a reactive carboxylic group, suitable reactive moieties on the other component can be amines and hydrazides (to form amide or N-acyl, N'-alkylhydrazide functions). For example, one may prepare a maleimide-derivatized phospholipid (e.g. phosphatidylethanolamine) which is then reacted with a mercaptoacetylated Fc-binding component (e.g. a protein, such as protein G), previously incubated in a deacetylation solution. As another example, one may prepare a maleimide-derivatized pegylated phospholipid (e.g. DSPE-PEG2000-maleimide) which is then reacted with a Fc-binding component (e.g. a protein, such as protein G), which has an accessible thiol function, brought by first reacting the protein with ((Sulfosuccinimidyl 6-[3'-(2-pyridyldithio)propionamido]-hexanoate) (Sulfo-LC-SDPD) and reducing the disulphide bond by adding tris(2-carboxy-ethyl)phosphine (TCEP).

Other excipients or additives may be present either in the dry formulation of the microbubbles or may be added together with the aqueous carrier used for the reconstitution thereof, without necessarily being involved (or only partially involved) in the formation of the stabilizing envelope of the microbubble. These include pH regulators, osmolality adjusters, viscosity enhancers, emulsifiers, bulking agents, etc. and may be used in conventional amounts. For instance compounds like polyoxypropylene glycol and polyoxyethylene glycol as well as copolymers thereof can be used. Examples of viscosity enhancers or stabilizers are compounds selected from linear and cross-linked poly- and oligosaccharides, sugars and hydrophilic polymers such as polyethylene glycol.

As the preparation of gas-filled microbubbles may involve a freeze drying or spray drying step, it may be advantageous to include in the formulation a lyophilization additive, such as an agent with cryoprotective and/or lyoprotective effect and/or a bulking agent, for example an amino-acid such as glycine; a carbohydrate, e.g. a sugar such as sucrose, mannitol, maltose, trehalose, glucose, lactose or a cyclodextrin, or a polysaccharide such as dextran; or a polyoxyalkyleneglycol such as polyethylene glycol.

The microbubbles of a composition according to the invention can be produced according to any known method in the art. Typically, the manufacturing method involves the preparation of a dried powdered material comprising an amphiphilic material as indicated above, preferably by lyophilization (freeze drying) of an aqueous or organic suspension comprising said material.

For instance, as described in WO 91/15244, film-forming amphiphilic compounds can be first converted into a lamellar form by any method employed for formation of liposomes. To this end, an aqueous solution comprising the film forming lipids and optionally other additives (e.g. viscosity enhancers, non-film forming surfactants, electrolytes etc.) can be submitted to high-speed mechanical homogenisation or to sonication under acoustic or ultrasonic frequencies, and then freeze dried to form a free flowing powder which is then stored in the presence of a gas. Optional washing steps, as disclosed for instance in US 5,597,549, can be performed before freeze drying.

According to an alternative embodiment (described for instance in US 5,597,549) a film forming compound and a hydrophilic stabiliser (e.g. polyethylene glycol, polyvinyl pyrrolidone, polyvinyl alcohol, glycolic acid, malic acid or maltol) can be dissolved in an organic solvent (e.g. tertiary butanol, 2-methyl-2-butanol or C₂Cl₄F₂) and the solution can be freeze-dried to form a dry powder.

Preferably, as disclosed for instance in International patent application WO2004/069284, a phospholipid (selected among those cited above and including at least one of the above-identified charged phospholipids) and a lyoprotecting agent (such as those previously listed, in particular carbohydrates, sugar alcohols, polyglycols, polyoxyalkylene glycols and mixtures thereof) can be dispersed in an emulsion of water with a water immiscible organic solvent (e.g. branched or linear alkanes, alkenes, cyclo-alkanes, aromatic hydrocarbons, alkyl ethers, ketones, halogenated hydrocarbons, perfluorinated hydrocarbons or mixtures thereof) under agitation. The emulsion can be obtained by submitting the aqueous medium and the solvent in the presence of at least one phospholipid to any appropriate emulsion-generating technique known in the art, such as, for instance, sonication, shaking, high pressure homogenization, micromixing, membrane emulsification, high speed stirring or high shear mixing. For instance, a rotor-stator homogenizer can be employed, such as Polytron^{®} PT3000. The agitation speed of the rotor-stator homogenizer can be selected depending from the components of the emulsion, the volume of the emulsion, the relative volume of organic solvent, the diameter of the vessel containing the emulsion and the desired final diameter of the microdroplets of solvent in the emulsion. Alternatively, a micromixing technique can be employed for emulsifying the mixture, e.g. by introducing the organic solvent into the mixer through a first inlet (at a flow rate of e.g. 0.05-5 mL/min), and the aqueous phase a second inlet (e.g. at a flow rate of 2-100 mL/min). Depending on the emulsion technique, the organic solvent can be introduced gradually during the emulsification step or at once before starting the emulsification step. Alternatively the aqueous medium can be gradually added to the water immiscible solvent during the emulsification step or at once before starting the emulsification step. Preferably, the phospholipid is dispersed in the aqueous medium before this latter is admixed with the organic solvent. Alternatively, the phospholipid can be dispersed in the organic solvent or it may be separately added the aqueous-organic mixture before or during the emulsification step. The so obtained microemulsion, which contains microdroplets of solvent surrounded and stabilized by the phospholipid material (and optionally by other amphiphilic film-forming compounds and/or additives), is then lyophilized according to conventional techniques to obtain a lyophilized material, which is stored (e.g. in a vial in the presence of a suitable gas) and which can be reconstituted with an aqueous carrier to finally give a gas-filled microbubbles suspension where the dimensions and size distribution of the microbubbles are substantially comparable with the dimensions and size distribution of the suspension of microdroplets.

A further process for preparing gas-filled microbubbles comprises generating a gas microbubble dispersion by submitting an aqueous medium comprising a phospholipid (and optionally other amphiphilic film-forming compounds and/or additives) to a controlled high agitation energy (e.g. by means of a rotor stator mixer) in the presence of a desired gas and subjecting the obtained dispersion to lyophilisation to yield a dried reconstitutable product. An example of this process is given, for instance, in WO97/29782, here enclosed by reference.

Spray drying techniques (as disclosed for instance in US 5,605,673) can also be used to obtain a dried powder, reconstitutable upon contact with physiological aqueous carrier to obtain gas-filled microbubbles.

The dried or lyophilized product obtained with any of the above techniques will generally be in the form of a powder or a cake, and can be stored (e.g. in a vial) in contact with the desired gas. The product is readily reconstitutable in a suitable physiologically acceptable aqueous liquid carrier, which is typically injectable, to form the gas-filled microbubbles, upon gentle agitation of the suspension. Suitable physiologically acceptable liquid carriers are sterile water, aqueous solutions such as saline (which may advantageously be balanced so that the final product for injection is not hypotonic), or solutions of one or more tonicity adjusting substances such as salts or sugars, sugar alcohols, glycols or other non-ionic polyol materials (eg. glucose, sucrose, sorbitol, mannitol, glycerol, polyethylene glycols, propylene glycols and the like).

According to an embodiment of the invention, the construct comprising the Fc-binding compound (i.e. a Fc-binding /envelope-forming construct or a Fc-binding/spacer/envelope-forming construct) can be admixed as such with the other components of the formulation, so to be incorporated into the stabilizing envelope upon reconstitution of the freeze-dried material obtained according to any of the above preparation methods.

Alternatively, the construct can be admixed as a suitably functionalized intermediate (e.g. a functionalized envelope-forming component such as a maleimide-containing phosphatidylethanolamine) to the initial formulation, to produce a freeze-dried material containing said intermediate; the Fc-binding component, containing a suitable complementary reactive moiety (e.g. thiol), can then be linked, by reacting the respective reactive moieties, to the intermediate compound already incorporated in the envelope of the reconstituted microbubbles.

In the case of the process disclosed in WO2004/069284, the construct containing the Fc-binding component can also be admixed with the components of the initial mixture, undergoing to the emulsion and lyophilisation steps. Alternatively, a micellar suspension containing the construct can be separately prepared and subsequently added to the already formed emulsion (containing the other film-forming components), preferably under heating. As above, instead of the formed construct, a functionalized intermediate can alternatively be used, which can then be reacted at any step of the process (e.g. in the emulsion phase or upon reconstitution of the lyophilized compound) with a Fc-binding component containing a complementary reactive moiety. According to an embodiment, a functionalized envelope-forming component (or envelope-forming/spacer intermediate construct) is added as a micellar suspension to the formed emulsion, under agitation. A compound comprising the Fc-binding component (containing the complementary reactive moiety) is then added to the obtained emulsion.

For example, one may add a micellar suspension of a maleimide derivative of an envelope-forming component (such as DSPE-maleimide or DSPE-PEG-maleimide) to the formed emulsion of film forming components. Then, a solution of a mercaptoacetylated Fc-binding component (e.g. protein G, 10 mg/mL in DMF), which has been incubated in deacetylation solution (50 mM sodium phosphate, 25 mM EDTA, 0.5 M hydroxylamine.HCl, pH 7.5) is added to the emulsion, under gentle agitation, before lyophilization of the emulsion. Alternatively, the emulsion containing the maleimide derivative of the envelope-forming component is lyophilized and then the mercaptoacetylad Fc-binding component is subsequently added to the reconstituted suspension of gas-filled microvesicles.

According to an alternative embodiment, the Fc-binding component can be incorporated into gas-filled microcapsules. Preferred examples of microcapsules are those having a stabilizing envelope comprising a polymer, preferably a biodegradable polymer, or a biodegradable water-insoluble lipid (such as tripalmitine) optionally in admixture with a biodegradable polymer. Examples of suitable microcapsules and of the preparation thereof are disclosed, for instance in US 5,711,933 and US 6,333,021, herein incorporated by reference in their entirety. Microcapsules having a proteinaceous envelope, i.e. made of natural proteins (albumin, haemoglobin) such as those described in US-A-4,276,885 or EP-A-0 324 938 (here incorporated by reference), can also be employed. The Fc-binding component can be incorporated into the microcapsules e.g. by binding it to an envelope-forming component of the microcapsules, according to the preparation methods illustrated above, or by admixing to the components forming the microcapsules envelope an amphiphilic component, as those previously illustrated, covalently bound to said Fc-binding component.

Any biocompatible gas, gas precursor or mixture thereof may be employed to fill the above microvesicles (hereinafter also identified as "microvesicle-forming gas").

The gas may comprise, for example, air; nitrogen; oxygen; carbon dioxide; hydrogen; nitrous oxide; a noble or inert gas such as helium, argon, xenon or krypton; a radioactive gas such as Xe¹³³ or Kr⁸¹; a hyperpolarized noble gas such as hyperpolarized helium, hyperpolarized xenon or hyperpolarized neon; a low molecular weight hydrocarbon (e.g. containing up to 7 carbon atoms), for example an alkane such as methane, ethane, propane, butane, isobutane, pentane or isopentane, a cycloalkane such as cyclobutane or cyclopentane, an alkene such as propene, butene or isobutene, or an alkyne such as acetylene; an ether; a ketone; an ester; halogenated gases, preferably fluorinated gases, such as or halogenated, fluorinated or prefluorinated low molecular weight hydrocarbons (e.g. containing up to 7 carbon atoms); or a mixture of any of the foregoing. Where a halogenated hydrocarbon is used, preferably at least some, more preferably all, of the halogen atoms in said compound are fluorine atoms.

Fluorinated gases are preferred, in particular perfluorinated gases, especially in the field of ultrasound imaging. Fluorinated gases include materials which contain at least one fluorine atom such as, for instance fluorinated hydrocarbons (organic compounds containing one or more carbon atoms and fluorine); sulfur hexafluoride; fluorinated, preferably perfluorinated, ketones such as perfluoroacetone; and fluorinated, preferably perfluorinated, ethers such as perfluorodiethyl ether. Preferred compounds are perfluorinated gases, such as SF₆ or perfluorocarbons (perfluorinated hydrocarbons), i.e. hydrocarbons where all the hydrogen atoms are replaced by fluorine atoms, which are known to form particularly stable microbubble suspensions, as disclosed, for instance, in EP 0554 213, which is herein incorporated by reference.

The term perfluorocarbon includes saturated, unsaturated, and cyclic perfluorocarbons. Examples of biocompatible, physiologically acceptable perfluorocarbons are: perfluoroalkanes, such as perfluoromethane, perfluoroethane, perfluoropropanes, perfluorobutanes (e.g. perfluoro-n-butane, optionally in admixture with other isomers such as perfluoro-isobutane), perfluoropentanes, perfluorohexanes or perfluoroheptanes; perfluoroalkenes, such as perfluoropropene, perfluorobutenes (e.g. perfluorobut-2ene) or perfluorobutadiene; perfluoroalkynes (e.g. perfluorobut-2-yne); and perfluorocycloalkanes (e.g. perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane and perfluorocycloheptane). Preferred saturated perfluorocarbons include, for example, CF₄, C₂F₆, C₃F₈, C₄F₈, C₄F₁₀, C₅F₁₂ and C₆F₁₂.

It may also be advantageous to use a mixture of any of the above gases in any ratio. For instance, the mixture may comprise a conventional gas, such as nitrogen, air or carbon dioxide and a gas forming a stable microbubble suspension, such as sulfur hexafluoride or a perfluorocarbon as indicated above. Examples of suitable gas mixtures can be found, for instance, in WO 94/09829, which is herein incorporated by reference. The following combinations are particularly preferred: a mixture of gases (A) and (B) in which the gas (B) is a fluorinated gas, selected among those previously illustrated, including mixtures thereof, and (A) is selected from air, oxygen, nitrogen, carbon dioxide or mixtures thereof. The amount of gas (B) can represent from about 0.5% to about 95% v/v of the total mixture, preferably from about 5% to 80%.

Particularly preferred gases are SF₆, C₃F₈, C₄F₁₀ or mixtures thereof, optionally in admixture with air, oxygen, nitrogen, carbon dioxide or mixtures thereof.

In certain circumstances it may be desirable to include a precursor to a gaseous substance (i.e. a material that is capable of being converted to a gas in vivo). Preferably the gaseous precursor and the gas derived therefrom are physiologically acceptable. The gaseous precursor may be pH-activated, photo-activated, temperature activated, etc. For example, certain perfluorocarbons may be used as temperature activated gaseous precursors. These perfluorocarbons, such as perfluoropentane or perfluorohexane, have a liquid/gas phase transition temperature above room temperature (or the temperature at which the agents are produced and/or stored) but below body temperature; thus, they undergo a liquid/gas phase transition and are converted to a gas within the human body.

For the use in MRI the microvesicles will preferably contain a hyperpolarized noble gas such as hyperpolarized neon, hyperpolarized helium, hyperpolarized xenon, or mixtures thereof, optionally in admixture with air, carbon dioxide, oxygen, nitrogen, helium, xenon, or any of the halogenated hydrocarbons as defined above.

For use in scintigraphy, the microvesicle will preferably contain radioactive gases such as Xe¹³³ or Kr⁸¹ or mixtures thereof, optionally in admixture with air, carbon dioxide, oxygen, nitrogen, helium, kripton or any of the halogenated hydrocarbons as defined above.

Once the gas-filled microvesicles comprising the Fc-binding component have been prepared, the desired component comprising the corresponding Fc-region to be bound can then be bound (through its Fc-region) to the microvesicles. Typically, the Fc-comprising component is dispersed in a physiologically acceptable liquid (e.g. saline solution) and then admixed to a suspension of gas-filled microvesicles in a physiologically acceptable liquid (e.g. also saline). The Fc-comprising component can be admixed in a relatively variable amount with respect to the microvesicles, in particular with respect to the amount of Fc-binding component present on the microvesicles. For instance, the molar ratio between the Fc-comprising component and the Fc-binding component in the suspension of microvesicles can vary from about 0.001/1 to about 100/1. Preferably, said ratio is from 0.01/1 to 20/1, more preferably from 0.1/1 to 10/1, and even more preferably from 0.5/1 to 5/1.

The so obtained microvesicles can be used as such or, if necessary, the mixture can undergo one or more washing steps, e.g. to remove the excess of Fc-comprising component. If desired, the obtained assembly can be further lyophilized and stored before use. In general, it is preferred to avoid having an excess of free (not bound) antibodies in the final suspension of microvesicles, as well as an excess of non-bound Fc-binding components. In one embodiment of the invention, the Fc-comprising component is added in a slightly defective stoichometric ratio with respect to the Fc-binding component (e.g. a molar ratio of about 0.9/1 of antibody/protein G).

If desired, the non-reacted Fc-binding components on the microvesicles can then be advantageously inactivated, to avoid possible binding of undesired Fc-comprising components, particularly when using the suspension in *in-vivo* diagnostic or therapeutic methods. For instance, the excess of non-reacted Fc-binding components can be inactivated by adding a convenient amount of a Fc-containing protein fragment (such as Human IgG Fc fragment or Goat IgG Fc fragment, both from Rockland Immunochemicals, Inc., Gilbertsville, PA, USA) to the suspension of gas-filled microvesicles associated with the Fc-comprising component.

According to alternative embodiments, the Fc-comprising component can be added to the preparation mixture at any suitable stage of the preparation. For instance, in the preparation method disclosed in WO2004/069284, the Fc-comprising component can be added into the emulsion containing the Fc-binding component, before lyophilization thereof.

As a further alternative, the Fc-comprising component can be admixed as a solid material to the lyophilized preparation of the microvesicles, before reconstitution thereof. The dry mixture will then, upon reconstitution with a physiologically acceptable carrier, form the desired assembly of microvesicles containing the Fc-comprising component.

Preferred Fc-comprising component are antibodies or chimeric proteins.

Examples of suitable antibodies which may be used for preparing gas-filled microvesicles according to the invention are listed in the following table 1, together with the respective biological targets or receptors

**Table 1: Antibodies for binding to Fc-binding components**

| **Antibody** | **Target** | **Comment/area of use** |
|---|---|---|
| Anti ICAM-1/CD54 | Intracellular Adhesion Molecule-1 | Endothelial cells activation |
| Anti ICAM-2 | Intracellular Adhesion Molecule-2 | Endothelial cells activation |
| Anti CD62L | L-Selectin | Endothelial cells activation |
| Anti CD62E | E-selectin | Endothelial cells activation |
| Anti CD62P | P-Selectin | Endothelial cells activation |
| Anti CD31 | PECAM-1 | Endothelial cells activation |
| Anti-TM/CD141 | Thrombomodulin | Endothelial cells activation |
| Anti-VCAM-1/CD106 | vascular cell adhesion molecule-1 | Endothelial cells activation |
| Anti CD105 | Endoglin | Marker of angiogenic endothelial cells |
| Anti Endocan | Endothelial cell specific molecule-1 (ESM-1) | As above |
| Anti-KDR/Flk-1 | Vascular endothelium growth factor Receptor-2 | As above |
| Anti-Flt-1 | Vascular endothelium growth factor Receptor-1 | As above |
| Anti-TEM1 | Tumor endothelial marker 1/endosialin | As above |
| Anti-TEM5 | Tumor endothelial marker 5 | As above |
| Anti-TEM7 | Tumor endothelial marker 7 | As above |
| Anti-TEM8 | Tumor endothelial marker 8 | As above |
| Anti-CD142 TF | Tissue Factor | As above |
| Anti-PSMA | Prostate Specific Membrane Antigen | As above |
| Anti-CXCR4(CD184) | CXCR4, Receptor for the CXC chemokine stromal derived Factor 1 | As above |
| Anti-Robo4 | Roundabout endothelial cell protein | As above |
| Anti-NRP1 | Neuropilin-1 | As above |
| Anti-integrin | Integrins, (Including e.g. VLA-1, VLA-2, VLA-3, VLA-4, VLA-5, VLA-6, α7 β1, αv β3, α5 β1, LFA-1, Mac-1, CD4la) | Endothelial cell marker |
| Anti-CD144 | VE-cadherin | Endothelial cell marker |
| Anti-vWF | von Willebrand factor | As above |
| Anti CD34 | CD34/ gp105-120 | As above |
| Anti-MadCam | Adressin | As above |
| Anti phosphatitylserine | Cell membrane phophatidylserine | Marker of apopotosis |
| Anti EDB fibronectin | Extra domain-B containing fibronectin | Marker of angiogenesis |
| Anti-CD44 | Cell adhesion molecules | Cell-cell interaction |
| Anti-CD14 | LPS receptor | Receptor of macrophages |
| Anti-TNF receptor1 | TNF receptor1 | Inflammation |
| Anti-PECAM | Platelet/endothelial cell | Cell-cell interaction |
| | adhesion molecule 1 | |
| Anti-CD41 | platelet glycoprotein (GPIIb/IIIa) integrin | Coagulation / thrombosis |
| Anti-C-Met | Hepatocyte Growth Factor Receptor | Marker of tumor growth |

The above antibodies preferably belong to the IgG (or "gamma immunoglobulins) isotype.

Examples of commercially available antibodies (identified by their biological target) are illustrated in the following table 2.

**Table 2: Commercial antibodies for binding to Fc-binding components**

| **TARGET** | **CLONE** | **HOST / Isotype** | **Species specif.** | **Sup.** | **Cat. #** |
|---|---|---|---|---|---|
| alphaVbeta3, CD51/61 complex | 23C6 | ms, IgG1 | hu, rb, not pig | S9 | AB2256 |
| alphaVbeta3, CD51/61 complex | LM609 | ms, IgG1 | hu, pg, rb | S9 | AB1976 |
| beta3/CD61 | F11 | ms, IgG1 | rt, (hu) | S20 | MCA1773 |
| beta3/CD61 | 25E11 | ms, IgM | hu | S9 | MAB1957B |
| CD 14 | B-A8 | ms, IgG1, k | hu | S20 | MCA660 B |
| CD 49e, VLA 5, Fibronectin receptor | SAM1 | ms, IgG2b | hu | S14 | 771 |
| CD106, VCAM-1 | MR106 | ms, IgG1, k | rt | S16 | 22681D |
| CD11b | OX42 | ms, IgG2a | rt | S20 | MCA275R |
| CD120a TNF-R1 | polyclonal (H-271) | rb, IgG | hu, ms, rt | S19 | sc-7895 |
| CD120a TNF-R1 | H-5 | ms, IgG2b | hu, ms, rt | S19 | sc-8436 |
| CD142 (tissue factor) | 4509 | ms, TgG1 | hu | S3 | 4509 |
| CD142 (tissue factor) | TF9-101H10 | ms, IgG1 | hu, pr | S8 | 612161 |
| CD15 | ZC18C or FMC10 | ms, IgM | hu | S9 | MAB1205F |
| CD163 (Macrophages) | ED2 | ms, IgG1 | rt | S2 | BM4001 |
| CD304 (neuropilin 1) | polyclonal (H-286) | rb, IgG | hu, rt, ms | S19 | sc-5541 |
| CD304 (neuropilin 1) | A-12 | ms, IgG1 | hu | S19 | sc-5307 |
| CD304 (neuropilin 1) | 130603 | ms, IgG2b | rt | S18 | MAB566 |
| CD309 (Flk-1) | A-3 | ms, IgG1 | rt, ms, hu | S19 | sc-6251 |
| CD309 (Flk-1) | 89B3A5 | rt, IgG2a | ms | S9 | MAB1669 |
| CD309 (Flk-1) | Avas12a1 | rt, IgG2a, k | ms | S11 | 14-5821 |
| CD309 (VEGF-R2) | KDR/EIC | ms, IgG1 | hu, (rt) | S1 | ab9530 |
| CD309 (VEGF-R2) | KDR-1 | ms, IgG1 | hu | S21 | V9134 |
| CD31 (PECAM-1) | TLD-3A12 | ms, IgG1 | rt (hu) | S20 | MCA1334G |
| CD31 (PECAM-1) | MEC 13.3 | rt, IgG2a, k | ms | S4 | 553370 |
| CD31 (PECAM-1) | 158-2B3 | ms, IgG1, k | hu | S15 | MS-654 |
| CD31 (PECAM-1) | JC/70A | ms, IgG1, k | hu | S15 | MS-353-P0 |
| CD35 (Complement receptor 1) | 8C12 | rt, IgG2a, κ | ms | S16 | 558768 |
| CD41 | P2 | ms, IgG1, k | hu | S14 | IMO718 |
| CD41 ((GPIIb-IIIa) complex) | 2Q948 | ms, IgG1 | hu | S22 | C2394-03A |
| CD41/61, GPIIbIIIa and anb3 | 7E3, Abciximab | Ms/Hu | hu | S12 | ReoPro |
| CD41/CD61 | CO 35E4 | ms, IgG1 | rb, go, sh | S20 | MCA1095 |
| CD45 Leukocyte common Ag, Ly-5 | 30-F11 | rt, IgG2b, k | ms | S11 | 12-0451 |
| CD45.2 | HIS41 | ms, IgG1 | rt | S11 | 14-0450 |
| CD45.2 | HIS41 | ms, IgG1 | rt | S11 | 12-0450 |
| CD51 (alphaV) | polyclonal | rb, Ig | hu, pg, ms, sh, gt | S9 | AB1930 |
| CD54 (ICAM-1) | 1A29 | ms, IgG1, k | rt | S16 | 22492D |
| CD54 (ICAM-1) | YN1/1.7.4 | rt, IqG2b, k | ms | S11 | 13-0541 |
| CD61 (integrin beta 3) | MHF4 | ms, IgG1 | hu, rt | S1 | ab20146 |
| CD62 (E-selectin) | 1.2B6 | ms, IgG1 | hu | S6 | M54180M |
| CD62P (P-Selectin) | AK4 | ms, IgG1 | hu, pg | S4 | 551345 |
| CD62P (P-Selectin)-IgG1 | RB40.34 | rt, IgG1, λ | ms | S4 | 553741 |
| CD62P, P-Selectin, GMP140.... | LYP20 | ms IgG1, k | hu, rt | S5 | 5111-P |
| CD62P-Selectin (C-20) | polyclonal | gt | hu, ms, rt | S19 | sc-6941 |
| c-Met | polyclonal (SP-260) | rb, IgG | ms, rt, (hu) | S19 | sc-162 |
| c-Met | polyclonal (C-28) | rb, IgG | hu, ms, rt | S19 | sc-161 |
| Complement C3b alpha | H206 | ms, IgG1 | hu | S17 | 61019 |
| Complement C3b-beta | H-11 | ms, IgG1 | hu | S17 | 61020 |
| D-dimer fibrin | DD-5 | ms, IgG1 | hu | S7 | 4440-0308 |
| Endothelin B receptor | polyclonal | rb, IgG | hu, ms, rt | S1 | ab1921 |
| FBP (folate receptor) | 42/033 | ms, IgG1 | bv | S7 | 4550-0238 |
| Fibrin | E8 | ms, IgG1 | hu, gp | S20 | MCA 707 |
| Fibrin D-dimer | B42.7.3 B6/22 | ms, TgG3, k | hu | S13 | MABH7 |
| Fibrin D-dimer, D-mono fibrin | DD-2 | ms, IgG1 ms, IgG1 | hu | S7 | 4440-0318 |
| Flk-1 (VEGF-R2 , CD309) | polyclonal | rb, IgG rb, IgG | hu, rt, ms | S19 | sc-504 |
| Flk-1/KDR/VEGF-R2 | polyclonal | rb, IgG | hu, ms, rt | S2 | DP076 |
| Flt-1/VEGF-R1 | polyclonal | rb, IgG | hu | S2 | DP077 |
| integrin alphaVbeta1, Fibronectin receptor | polyclonal | go, Ig | hu | S9 | AB1950 |
| Macrophage C57/BL | F4/80 (cl : A3-1) | rt, IgG2b | ms | S20 | MCA 497 B |
| Macrophage marker | Hsn 7D2 | ms, IgG1 | hu | S15 | MS-618-P |
| Macrophages | Ki-M2R | ms, IgG1 | rt | S2 | BM4003 |
| Macrophages CD172a | ED9 | ms, IgG1 | rt | S20 | MCA620 |
| Monocytes/ macrophages | ED1 | ms, IgG1 | rt | S2 | BM4000 |
| PSMA | Y-PSMA1 | ms, IgG2b, k | hu | S2 | DM1037 |
| PSMA | polyclonal | rb, IgG | hu | S1 | ab22335 |
| PSMA (C-Terminal) ZMD.80 | polyclonal | rb, Ig | hu | S23 | 344100 |
| ratVEGF164 | polyclonal | gt, IgG | rt, m s, hu | S18 | AF564 |
| skeletal myosin | MY-32 | ms, IgG1 | hu, rb, rt, ms | S23 | 08-0105 |
| VEGF-R2 (Flk-1/KDR) | 89106 | ms, IgG1 | hu, not ms | S18 | MAB3572 |
| Von Willebrand/Factor VIII | polyclonal | rb, | hu, ms, cow, horse | S10 | A 0082 |
| Von Willebrand/Factor VIII | A0082 | rb | hu | S10 | U 0034 |

| | | | | | |
|---|---|---|---|---|---|
| **Species abbreviations:** bv= bovine, ch= chicken, dg= dog, gt= goat, gp= guinea pig, hu= human, ms= mouse, pg= pig, pr= primate, rb= rabbit, rt= rat, sh= sheep, **Suppliers abbreviations:** S1= Abcam, Cambridge, UK, S2= Acris Antibodies GmbH, Hiddenhausen, Germany, S3= American diagnostica, Stamford, CT, USA, S4= BD Biosciences, San Jose, CA USA, S5= BioCytex, Marseille, France, S6= Biodesign International, Saco, Maine, USA, S7= Biogenesis, Poole, UK, S8= Calbiochem, a brand of EMD chemicals, San Diego, CA, USA, S9= Chemicon, Chemicon is Millipore company, Billerica, MA, USA, S10= Dako, Glostrup, Denmark, S11= eBioscience, San Diego, CA, USA, S12= Eli Lilly, Indianapolis, IN, USA, S13= Endotell, Allschwil, Switzerland, S14= Immunotech, Marseille, France, S15= NeoMarkers, Freemont, CA, USA, S16= Pharmingen, San Diego, CA, USA, S17= Progen, Ballwin, MO, USA, S18= R&D system, Minneapolis, MN, USA , S19= Santa Cruz Biotechnology, Santa Cruz, CA, USA, S20= Serotec, AbD Serotec, Raleigh, NC, USA, S21= Sigma, Sigma-Aldrich, Buchs, Switzerland, S22= USBiological, Swampscott, MA, USA, S23= Zymed, South San Francisco, USA. | | | | | |

Preferred gas-filled microvesicles of the invention are those containing protein G (as Fc-binding component) and an IgG antibody (e.g. selected among those listed above) bound thereto.

Suitable chimeric proteins include recombinant fusion proteins containing a Fc-region of an antibody and at least one protein, or antibody Fab fragment, of interest. For example, a recombinant molecule resulting from fusion of P-selectin glycoprotein ligand (PSGL) and human IgG1, and acting as an antagonist of P-selectin, is disclosed in PCT application WO 98/42750, herein incorporated by reference. Etanercept, a chimeric protein available under the trade name Enbrel® (Amgen, Newbury Park, Thousand Oaks, CA, USA), is made from the combination of two naturally occurring soluble human 75-kilodalton TNF receptors, linked to an Fc portion of an IgG1. Infliximab, a chimeric monoclonal antibody commercialized under the trade name Remicade® (Centocor Inc., Horsham, PA, USA) is made from the combination of murine binding VK and VH domains and human constant Fc domains and is described to block the action of TNFalpha.

According to an embodiment of the invention, the gas-filled microvesicles comprise one single type of component comprising a Fc-region of an antibody.

According to an alternative embodiment, the microvesicles of the invention may comprise two or more different types of components comprising a Fc-region of an antibody, e.g. capable of binding to different biological targets so to obtain bi- or multi-specific targeted microvesicles. Accordingly, a microvesicle comprising a component comprising a Fc-region of an antibody, may comprise one or more different types of components comprising a Fc-region of an antibody.

A contrast agent according to the invention is preferably stored in dried powdered form and as such can advantageously be packaged in a diagnostic and/or therapeutic kit. The kit may comprise, for instance, a first container, containing the lyophilized composition in contact with a selected microvesicle-forming gas (as those previously discussed) and a second container, containing a physiologically acceptable aqueous carrier. Said two component kit can include two separate containers or a dual-chamber container. In the former case the container is preferably a conventional septum-sealed vial, wherein the vial containing the lyophilized residue is sealed with a septum through which the carrier liquid may be injected using an optionally pre-filled syringe. In such a case the syringe used as the container of the second component is also used then for injecting the contrast agent. In the latter case, the dual-chamber container is preferably a dual-chamber syringe and once the lyophilisate has been reconstituted and then suitably mixed or gently shaken, the container can be used directly for injecting the contrast agent.

According to a preferred embodiment, a kit of the invention comprises:
- a first container, comprising gas-filled microvesicles, or precursors thereof, comprising a first component having binding affinity for the Fc-region of an antibody; and
- a second container comprising a second component comprising an Fc-region capable of binding to said first component through said Fc-region.

Preferably, the first container comprises a precursor of gas-filled microvesicles in powdered dry form, in contact with a microvesicle-forming gas.

The second component can be present in the container in dry solid form or as a suspension in a physiologically acceptable aqueous carrier.

The above kit can optionally contain a physiologically acceptable aqueous carrier (either in a separate container or in a dual chamber container, as previously illustrated), for reconstitution of the dry components before injection.

The gas-filled microvesicles of the invention are preferably prepared by first adding a physiologically acceptable aqueous carrier to the powdered precursor of the gas-filled microvesicles, in contact with the desired gas, under gentle agitation. The second component is then added to the suspension of microvesicles, either in solid form or as a liquid suspension, under gentle agitation.

The contrast agents of the present invention may be used in a variety of *in-vivo* and *in-vitro* diagnostic and/or therapeutic imaging methods, including in particular ultrasound and magnetic resonance imaging.

Typically, a patient is administered an effective amount of the contrast agent (e.g. by injection) and the body part or tissue to be imaged or treated is subjected to ultrasound scanning to image or treat said body part or tissue. The term patient includes any subject undergoing to the administration of the contrast agent, either for diagnostic/therapeutic purposes or for experimental purposes (including, for instance, use of a contrast agent in laboratory animals, e.g. to follow an experimental therapeutic treatment).

Diagnostic imaging includes any contrast enhanced imaging of a body part or tissue, as well as any other diagnostic technique or method such as, for instance, quantification diagnostic techniques (including e.g. blood pressure, flow and/or perfusion assessment).

Therapeutic imaging includes within its meaning any method for the treatment of a disease in a patient which comprises the use of a contrast imaging agent (e.g. for the delivery of a therapeutic compound to a selected receptor or tissue), and which is capable of exerting or is responsible to exert a biological effect in vitro and/or in vivo. Therapeutic imaging may advantageously be associated with the controlled localized destruction of the gas-filled microvesicles, e.g. by means of ultrasound waves at high acoustic pressure (typically higher than the one generally employed in non-destructive diagnostic imaging methods). This controlled destruction may be used, for instance, for the treatment of blood clots (a technique also known as sonothrombolysis), optionally in combination with the release of a suitable therapeutic compound associated with the contrast agent. Alternatively, said therapeutic imaging may include the delivery of a therapeutic compound into cells, as a result of a transient membrane permeabilization at the cellular level induced by the localized burst of the microvesicles. This technique can be used, for instance, for an effective delivery of genetic material into the cells; optionally, a drug can be locally delivered in combination with genetic material, thus allowing a combined pharmaceutical/genetic therapy of the patient (e.g. in case of tumor treatment).

A variety of imaging techniques may be employed in ultrasound applications, for example including fundamental and harmonic B-mode imaging, pulse or phase inversion imaging and fundamental and harmonic Doppler imaging; if desired three-dimensional imaging techniques may be used. Furthermore, diagnostic techniques entailing the destruction of gas-filled microvesicles (e.g. by means of ultrasound waves at high acoustical pressure) are also contemplated, for instance in methods for assessing blood perfusion. Microvesicles according to the invention can typically be administered in a concentration of from about 0.01 to about 5.0 µl of gas per kg of patient, depending e.g. on their respective composition, the tissue or organ to be imaged and/or the chosen imaging technique. This general concentration range can of course vary depending from specific imaging applications, e.g. when signals can be observed at very low doses such as in color Doppler or power pulse inversion. Possible other diagnostic imaging applications include scintigraphy, light imaging, and X-ray imaging, including X-ray phase contrast imaging.

In addition, the gas-filled microvesicles of the invention can also be used in *in-vitro* tests. For instance, gas-filled microvesicles having Fc-binding components are useful for selecting new antibodies for selected target proteins. A particular *in-vitro* test comprises incubating microvesicles with attached antibodies in suspension over a layer of target protein. After incubation, subsequent washing of excess microvesicles, the attachment of the microvesicles to the target (indicative of the attachment of antibodies to the target) can easily be visualized and quantified by microscopic observation. In this way, such test can give evidence of antibody affinity for a particular target. Such test can be used to select an antibody among a set of antibodies for a particular target.

Another *in-vitro* test comprises using antibody-bearing microvesicles (with the antibodies attached to the vesicles by their Fc portion) to assess the avidity of a multivalent construct (namely the microvesicles with the antibodies) in comparison to free antibodies in a competition setup. This competition setup consists of incubating antibody-bearing microvesicles concomitantly with free antibodies at different concentrations in different wells coated with a protein of interest of a multi-well plate. This test enables to determine the concentration required to displace the microbubbles from the target protein coating. As in the previous in vitro test, binding of microvesicles is assessed by microscopic observation of the microvesicles at the surface of the protein coating.

Another in vitro test consists of feeding a suspension of antibody-bearing microvesicles in a flow cell coated with a protein of interest. Binding of microvesicles to the target protein can be assessed by microscopic observation as described above. This in vitro test is useful to determine attachment of targeted microvesicles on a target protein in flow conditions corresponding more or less to the shear stress observed in arteries or blood vessels.

The following examples will help to further illustrate the invention.

### Examples

**Table 3: Materials used in the examples**

| **Abbreviation** | **Full name** | **Supplier/Catalog#** |
|---|---|---|
| DSPC | 1,2-distearoyl-sn-glycero-3 phosphocholine | Genzyme Pharmaceuticals (Liestal - Switzerland) # |
| | | LP-04-013 |
| Palmitic acid | | Fluka (Buchs - Switzerland) #76120 |
| DPPG | 1,2-dipalmitoyl-sn-glycero-3 phosphoglycerol, sodium salt | Genzyme Pharmaceuticals # LP-04-016 |
| DSPE-PEG2000-mal | 1,2-Distearoyl-sn-Glycero-3-Phosphoethanolamine-N-[Maleimide(Polyethylene Glycol)2000] (Ammonium Salt) | Avanti Polar Lipids (Alabaster, AL, USA) #880126 |
| Protein G | Recombinant protein G | BioVision (Mountain View, CA, USA) #6510-5 |
| Sulfo-LC-SPDP | Sulfosuccinimidyl 6-[3'-(2-pyridyldithio)propionamido]-hexanoate | Pierce (Rockford, IL,USA) #21650 |
| TCEP | Tris(2-Carboxyethyl) phosphine Hydrochloride | Pierce #20490 |
| Tris HCl | Tris (hydroxymethyl) aminomethane hydrochloride | Fluka #93358 |
| PEG4000 | Polyethylene glycol 4000 | Fluka #81240 |
| DPPE-PEG5000 | N-(Carbonyl-methoxypolyethyleneglycol 5000)-1,2-dipalmitoyl-sn-glycero-3-phosphoethanolamine, sodium salt | Genzyme Pharmaceuticals # LP-R4-075 |
| Rat IgG antibody | Rat IgG whole molecule | Rockland (Gilbertsville, PA, USA) #012-0102 |
| Goat IgG antibody | Goat IgG whole molecule | Rockland #005-0102 |
| goat anti rat Fc antibody | Goat anti-Rat IgG, Fc | Chemicon (Temecula, CA, USA) #AP138 |
| rat anti-mouse P-selectin antibody | Purified NA/LE Rat Anti-Mouse CD62P | BD Biosciences (Franklin Lakes, NJ USA) #553741 |

### Example 1

### Preparation of gas-filled microvesicles containing protein G

(i) 80 mg of a mixture of DSPC/DPPG/ (41/34/25 by moles) were dissolved in cyclooctane (6.4 mL) at 70°C. The organic phase containing the phospholipids was then added to the aqueous phase (PEG4000 10% in distilled water - 80mL) and emulsified by using a high speed homogenizer (Megatron MT3000) for 5 min (11000 rpm), to obtain an emulsion.
(ii) In a separate vessel, 8.7 mg of DSPE-PEG2000-mal were dissolved in ethanol (0.4 mL); after solvents evaporation, the lipid film obtained was dried overnight at 25°C and 0.2 mBar and dispersed in 550 µL of phosphate buffer 100 mM pH 6.0 at 60°C, to obtain a micellar suspension of DSPE-PEG2000-mal.
(iii) The micellar suspension was added to 75 mL of the previous emulsion (40 nmoles maleimide/ mL emulsion) and the resulting emulsion was heated under stirring at 60°C for 1 h, then cooled at room temperature (about 22°C).
(iv) An aqueous suspension of protein G (300 µL of a 5 mg/mL stock solution, i.e. 57.7 nmoles) was reacted with 18 µL of a 10 mM Sulfo-LC-SDPD solution in 182 µL of 50 mM phosphate buffer 150 mM NaCl pH 7.4, for 40 min at room temperature. The solution was spun through a 2 mL spin-column at 1000 g (Zeba spin column, Pierce, #89889) equilibrated in phosphate buffer 5 mM pH 7.4. The functionalized protein G was then reduced with 1 mM TCEP, 50 mM Tris HCl/5 mM EDTA pH 6.8, for 15 min at room temperature. The reduced protein G was spun through a 2 mL spin-column at 1000 g.
(v) 170 mL of the solution containing the reduced protein G (at a concentration of about 2.2 mg/mL - 85 nmoles/mL) were added to 10 mL of the emulsion and the resulting mixture was agitated at 22°C for 2 h. The obtained emulsion was finally diluted twice in 20 mL of 10% PEG4000 solution sampled in DIN4R vials (300 µL per vial).
(vi) Vials were frozen at -50 °C for 2 h (Christ Epsilon lyophilizer), then freeze-dried at -25°C and 0.2 mBar. The lyophilized product was then exposed to an atmosphere containing 35% of perfluoro-n-butane and 65% of air. The vials were sealed.
(vii) The product was dispersed in a volume of saline (1 mL, 150 mM NaCl) by gentle hand shaking. The suspension of microbubbles was washed twice (centrifuged at 180 g, 10 min) and the supernatant containing the microbubbles was resuspended in 1 mL of saline. Number and volume of microbubbles were determined by Coulter counter measurement, and the total surface of the microbubbles was calculated.

### Example 2

### Preparation of gas-filled microvesicles containing protein G and DPPE-PEG5000

Microbubbles are prepared according the example 1 with the following modifications.

After performing step (iv) of example 1, for grafting the protein G, a solution of DPPE-PEG5000 in micellar form (1.28 mg in 130 µL distilled water - 1.5% molar ratio) was added to the emulsion, together with 140 mL of the solution containing the reduced protein G. The resulting emulsion was heated under stirring at 50°C for 1 h, then cooled at room temperature (about 22°C). The obtained emulsion was finally diluted twice in 20 mL of 10% PEG4000 solution sampled in DIN4R vials (300 µL per vial).

Vials were then treated according to steps (vi) to (vii) of example 1.

### Example 3

### Attachment of rat and goat IgG antibody onto microvesicles containing protein G

Non specific Rat IgG (Rockland - #012-0102) and non specific goat IgG (Rockland - #005-0102) were used.

300 µL of a solution with different amounts of antibody (see tables 4 and 5) were added into vials containing suspensions of protein G-microvesicles prepared according to example 1 and 2, respectively. Traces of ¹²⁵I-labeled antibody were added to the solution of the unlabeled antibody, for the subsequent determination of the antibody density.

After 10 min under agitation, the suspension of microbubbles was washed twice (centrifuged at 180 g, 10 min) and the supernatant containing the microbubbles was resuspended in 1 mL of saline. Number and volume of microbubbles were determined by Coulter counter measurement, and the total surface of the microbubbles was calculated.

The density of the antibody bound to the surface of the microbubbles was then determined by measuring the radioactive response of the suspension (by means of Auto-Gamma Cobra II - Packard), converting said value into the corresponding number of molecules of antibody and dividing said number by the total surface of the microbubbles, as determined by the Coulter counter measurement.

**Table 4: Density of antibody on the surface of microvesicles from example 1**

| Antibody (µg/vial) | Density Rat IgG (molecules/µm²) | Density Goat IgG (molecules/µm²) |
|---|---|---|
| 10 | 2093 | 3791 |
| 20 | 3909 | 8440 |
| 40 | 5754 | 8080 |

**Table 5: Density of antibody on the surface of microvesicles from example 2**

| Antibody (µg/vial) | Density Rat IgG (molecules/µm²) | Density Goat IgG (molecules/µm²) |
|---|---|---|
| 10 | na | na |
| 20 | 1820 | 2864 |
| 40 | na | 5671 |

| | | |
|---|---|---|
| na= not available | | |

### Example 4

### Preparation of microbubbles containing Goat anti rat Fc antibody

80 mg of a mixture of DSPC/DPPG/Palmitic acid (41/34/25 by moles) were dissolved in cyclooctane (6.4 mL) at 70°C. The organic phase containing the phospholipids was then added to the aqueous phase (PEG4000 10% in distilled water - 80 mL) and emulsified by using a high speed homogenizer (Megatron MT3000) for 5 min (11000 rpm), to obtain an emulsion.

In a separate vessel, 8.7 mg of DSPE-PEG2000-mal were dissolved in ethanol (0.4 mL); after solvents evaporation, the lipid film obtained was dried overnight at 25°C and 0.2 mBar and dispersed in 550 µL of phosphate buffer 100 mM pH 6.0 at 60°C, to obtain a micellar suspension of DSPE-PEG2000-mal.

The micellar suspension was added to 75 mL of the previous emulsion (40 nmoles maleimide/ mL emulsion) and the resulting emulsion was heated under stirring at 60°C for 1 h, then cooled at room temperature (about 22°C).

A water suspension of goat anti rat Fc antibody (1 mL of a 2 mg/mL stock solution, i.e. 13.33 nmoles) was reacted with 6.7 µL of a 10 mM Sulfo-LC-SDPD solution in 400 µL of 50 mM phosphate buffer 150 mM NaCl pH 7.4, for 40 min at room temperature. The solution was spun through a 5 mL spin-column at 1000 g (Zeba spin column, Pierce, #89891) equilibrated in phosphate buffer 5 mM pH7.4. The functionalized goat anti rat Fc antibody was then reduced with 1 mM TCEP, 50 mM Tris HCl/5 mM EDTA pH 6.8, for 15 min at room temperature. The reduced goat anti rat Fc antibody was spun through a 5 mL spin-column at 1000 g.

1.3 mL of the solution containing the reduced antibody (at a concentration of about 1,05 mg/mL (or 7 nmoles/mL)) was added to 10 mL of the emulsion and the resulting mixture was agitated at 22°C for 2 h. The obtained emulsion was finally diluted twice in 20 mL of 10% PEG4000 solution sampled in DIN4R vials (300 µL per vial).

Vials were frozen at -50 °C for 2 h (Christ Epsilon lyophilizer), then freeze-dried at -25°C and 0.2 mBar. The lyophilized product was then exposed to an atmosphere containing 35% of perfluoro-n-butane and 65% of air. The vials were sealed.

The product was dispersed in a volume of saline (1 mL, 150 mM NaCl) by gentle hand shaking. The suspension of microbubbles was washed twice (centrifuged at 180 g, 10 min) and the supernatant containing the microbubbles was resuspended in 1 mL of saline. Number and volume of microbubbles were determined by Coulter counter measurement, and the total surface of the microbubbles was calculated.

### Example 5

### Attachment of rat antibody onto microvesicles containing Goat anti rat Fc antibody

Non specific Rat IgG antibody was used.

300 µL of a solution with different amounts of antibody (see table 6) were added into various vials containing suspensions of microvesicles prepared according to example 4. Traces of ¹²⁵I-labeled antibody were added to the solution of the unlabeled antibody, for the subsequent determination of the density of the antibody.

After 10 minutes under agitation, the suspension of microvesicles was washed twice (centrifuge at 180 g, 10 min) and the supernatant containing the microbubbles was resuspended in 1 mL of saline. Number and volume of microbubbles were determined by Coulter counter measurement, and the total surface of the microbubbles was calculated.

The density of the antibody bound to the surface of the microbubbles was then determined by measuring the radioactive response of the suspension (by means of Auto-Gamma Cobra II - Packard), converting said value into the corresponding number of molecules of antibody and dividing said number by the total surface of the microbubbles, as determined by the Coulter counter measurement.

**Table 6: Density of antibody on the surface of the Goat anti rat Fc antibody - microbubbles**

| Example | Antibody (µg/vial) | Density Rat IgG (molecules/µm²) |
|---|---|---|
| 5a | 10 | 3670 |
| 5b | 20 | 4971 |
| 5c | 40 | 6850 |

When the experiment was repeated, for comparative purpose, with non specific goat IgG (having an Fc-region not specifically recognized by the Goat anti rat Fc antibody), the density of the antibody on the surface of the microvesicles was negligible.

### Example 6

### Preparation of microvesicles containing protein G bound to anti P-selectin antibody

20 µL (20 µg of antibody) of a solution of a rat anti-mouse P-selectin antibody (BD Biosciences #553741) was added in 280 µL of saline.

A vial containing suspensions of protein G -microvesicles prepared according to example 1 was dispersed in a volume of saline (0.7 mL, 150 mM NaCl) by gentle hand shaking.

Then the antibody solution was added into the vial. The microbubble suspension was agitated (on a wheel) for 10 minutes before use.

### Example 7

### In vitro binding activity of microvesicles containing protein G bound to rat anti-mouse P-selectin antibody

To test the effective binding of these conjugates, targeted microvesicles prepared according to example 6 were injected in a flow chamber set up comprising a coating of Mouse P-Selectin (CD62P-Fc Chimera, from R&D Systems (Minneapolis, MN, USA). Microvesicles (at equivalent surface of 5 x 10⁹ µm²/14 mL) were drawn through the flow chamber (FCS2, Bioptech, USA) and their adhesion onto the mouse P-selectin coating layer was assessed for 10 min at a flow rate of 1.0 mL/min (shear rate of 114 s⁻¹) in the presence of 50% human plasma in PBS (v:v, Biomeda collected on citrate, ref. ES1020P, Stehelin & Cie AG). A quantitative analysis of microvesicles accumulation was performed by counting the number of microvesicles adhering in the observed area at 2 min intervals over the total 10 min infusion, using the image processing program Analysis FIVE (SIS, Germany). After 10 min, five pictures were taken randomly and averaged then divided by ten, representing the rate of microvesicles accumulation per minute (RMA/min). Each observed area was 183 x 137 µm, as measured with the aid of a stage micrometer. Imaging was performed between the middle and the exit of the chamber.

A mean value of 5.28 RMA/min was determined.

Comparative tests with control isotype non-binding antibody gave a mean value of 0.04 RMA/min.

### Example 8

### Preparation of microvesicles containing Goat anti-rat Fc antibody bound to rat anti-mouse P-selectin antibody

10 µL (10 µg of antibody) of a solution of a rat anti-mouse P-selectin antibody (BD Biosciences #553741) was added in 290µL of saline.

A vial containing goat anti rat Fc antibody microvesicles prepared according to example 4 was dispersed in a volume of saline (0.7 mL, 150 mM NaCl) by gentle hand shaking.

Then the antibody solution was added into the vial. The microbubble suspension was agitated (on a wheel) for 10 min before use.

### Example 9

### In vitro binding activity of microvesicles containing Goat anti-rat Fc antibody bound to rat anti-mouse P-selectin antibody

To test the effective binding of these conjugates, targeted microvesicles prepared according to example 8 were injected in a flow chamber set up comprising a coating of Mouse P-Selectin (CD62P-Fc Chimera, from R&D Systems (Minneapolis, MN, USA). Microvesicles (at equivalent surface of 5 x 10⁹ µm²/14 mL) were drawn through the flow chamber (FCS2, Bioptech, USA) and their adhesion onto the P-selectin coating layer was assessed for 10 min at a flow rate of 1.0 mL/min (shear rate of 114 s⁻¹) in the presence of 50% human plasma in PBS (v:v, Biomeda collected on citrate, ref. ES1020P, Stehelin & Cie AG). A quantitative analysis of microvesicle accumulation was performed by counting the number of microvesicles adhering in the observed area at 2 min intervals over the total 10 min infusion, using the image processing program Analysis FIVE (SIS, Germany). After 10 min, five pictures were taken randomly and averaged then divided by ten, representing the rate of microvesicle accumulation per minute (RMA/min). Each observed area was 183 x 137 µm, as measured with the aid of a stage micrometer. Imaging was performed between the middle and the exit of the chamber.

A mean value of 6.41 RMA/min was determined.

Comparative tests with control isotype non-binding antibody gave a mean value of 0.08 RMA/min.

## Claims

1. Gas-filled microvesicle, comprising a boundary envelope containing said gas, wherein said microvesicle comprises:
- a first component, bound to said envelope, having binding affinity for a Fc-region of an antibody; and
- a second component comprising a Fc-region of an antibody, bound to said first component through said Fc-region, said second component comprising a targeting ligand or a therapeutic agent.

2. Gas-filled microvesicle according to claim 1 wherein the second component is an antibody or a chimeric protein.

3. Gas-filled microvesicles according to claim 1 wherein said first component is a protein, an anti-Fc antibody or a Fc-receptor.

4. Gas-filled microvesicles according to claim 3 wherein said protein is selected from the group comprising natural or recombinant protein G, protein A and recombinant fusion protein A/G.

5. Gas-filled microvesicle according to any of the previous claims wherein said first component is covalently bound to said envelope.

6. Gas-filled microvesicle according to claim 5, wherein the first component is bound to an amphiphilic compound included in the envelope of the microvesicle.

7. Gas-filled microvesicle according to claim 6, wherein said amphiphilic compound is a phospholipid, optionally comprising a hydrophilic polymer.

8. Gas-filled microvesicle according to claim 7, wherein said phospholipid is phosphatidylethanolamine.

9. Gas-filled microvesicle according to any of the preceding claims, wherein the microvesicles are microbubbles comprising a phospholipid in the boundary envelope.

10. Gas-filled microvesicle according to any of the preceding claims, wherein the gas is selected from air, nitrogen, oxygen, carbon dioxide, hydrogen, nitrous oxide, noble or inert gas, a radioactive gas, a hyperpolarized noble gas, a low molecular weight hydrocarbon, an ether, a ketone, an ester, a halogenated gas or mixtures thereof.

11. Gas-filled microvesicle according to claim 10 wherein the gas is a fluorinated gas, optionally in admixture with nitrogen or air.

12. A suspension comprising a plurality of gas filled microvesicles as defined in any of the previous claims, dispersed in a physiologically acceptable aqueous carrier.

13. A pharmaceutical kit comprising:
- a precursor of a gas-filled microvesicle, comprising: (i) a first component having binding affinity for a Fc-region of an antibody, and (ii) a second component, comprising a Fc-region capable of binding to said first component through said Fc-region, said precursor being in the form of a dry lyophilized composition in contact with a gas, and
- a physiologically acceptable aqueous carrier.

14. A pharmaceutical kit comprising:
- a gas-filled microvesicle, or a precursor thereof, comprising a first component having binding affinity for a Fc-region of an antibody, and
- a second component, comprising a Fc-region capable of binding to said first component through said Fc-region.

15. A pharmaceutical kit according to claim 14 comprising:
- a first container, comprising a gas-filled microvesicle, or precursor thereof, comprising a first component having binding affinity for the Fc-region of an antibody; and
- a second container comprising a second component comprising an Fc-region capable of binding to said first component through said Fc-region.

16. A pharmaceutical kit according to claim 15 wherein said first container comprises a precursor of said gas-filled microvesicle in powdered dry form, in contact with a gas.

17. A pharmaceutical kit according to claim 15 wherein said second container comprises said second component in dry solid form or as a suspension in a physiologically acceptable aqueous carrier.

18. A pharmaceutical kit according to any of the previous claims 14 to 17, further comprising a physiologically acceptable aqueous carrier.

19. A method for preparing a suspension of gas-filled microvesicles as defined in claim 12 which comprises:
- preparing a suspension of gas-filled microvesicles in a physiologically acceptable aqueous carrier, said microvesicles comprising a first component having binding affinity for a Fc-region of an antibody; and
- admixing to said suspension a second component, comprising a targeting ligand or a therapeutic agent, and comprising a Fc-region of an antibody capable of binding to said first component.

## Patentansprüche

1. Gasgefülltes Mikrovesikel, das eine Umhüllung aufweist, die das Gas enthält, wobei das Mikrovesikel
- eine erste Komponente, die an die Umhüllung gebunden ist und Bindungsaffinität für einen Fc-Bereich eines Antikörpers aufweist, und
- eine zweite Komponente enthält, die einen Fc-Bereich eines Antikörpers enthält und die über diesen Fc-Bereich an die erste Komponente gebunden ist, wobei die zweite Komponenten einen zielführenden Liganden oder ein therapeutisches Mittel enthält.

2. Gasgefülltes Mikrovesikel nach Anspruch 1, wobei die zweite Komponente ein Antikörper oder ein chimäres Protein ist.

3. Gasgefüllte Mikrovesikel nach Anspruch 1, wobei die erste Komponente ein Protein, ein Anti-Fc-Antikörper oder ein Fc-Rezeptort ist.

4. Gasgefüllte Mikrovesikel nach Anspruch 3, wobei das Protein aus der Gruppe ausgewählt ist, die natürliches oder rekombinantes Protein G, Protein A und rekombinantes Fusionsprotein A/G enthält.

5. Gasgefülltes Mikrovesikel nach einem der vorhergehenden Ansprüche, wobei die erste Komponente kovalent an die Umhüllung gebunden ist.

6. Gasgefülltes Mikrovesikel nach Anspruch 5, wobei die erste Komponente an eine amphiphile Verbindung gebunden ist, die in der Umhüllung des Mikrovesikels enthalten ist.

7. Gasgefülltes Mikrovesikel nach Anspruch 6, wobei die amphiphile Verbindung ein Phospholipid ist, das optional ein hydrophiles Polymer enthält.

8. Gasgefülltes Mikrovesikel nach Anspruch 7, wobei das Phospholipid Phosphatidylethanolamin ist.

9. Gasgefülltes Mikrovesikel nach einem der vorhergehenden Ansprüche, wobei die Mikrovesikel Mikrobläßchen sind, die ein Phospholipid in der Umhüllung enthalten.

10. Gasgefülltes Mikrovesikel nach einem der vorhergehenden Ansprüche, wobei das Gas aus Luft, Stickstoff, Sauerstoff, Kohlendioxid, Wasserstoff, Stickstoffoxid, Edel- oder Inertgas, einem radioaktiven Gas, einem hyperpolarisierten Edelgas, einem Kohlenwasserstoff mit niedrigem Molekulargewicht, einem Ether, einem Keton, einem Ester, einem halogenierten Gas oder Mischungen davon ausgewählt ist.

11. Gasgefülltes Mikrovesikel nach Anspruch 10, wobei das Gas ein fluoriertes Gas ist, ggf. in Mischung mit Stickstoff oder Luft.

12. Suspension, die eine Vielzahl gasgefüllter Mikrovesikel wie in einem der vorhergehenden Ansprüche definiert dispergiert in einem physiologisch verträglichen, wässrigen Träger enthält.

13. Pharmazeutisches Kit, das
- eine Vorläufer eines gasgefüllten Mikrovesikels, der (i) eine erste Komponente mit Bindungsaffinität für einen Fc-Bereich eines Antikörpers und (ii) eine zweite Komonente enthält, die einen Fc-Bereich enthält, der über den Fc-Bereich an die erste Komponente binden kann, wobei der Vorläufer in Form einer trocknen, lyophilisierten Zusammensetzung vorliegt, die in Kontakt mit einem Gas steht, und
- einen physiologisch verträglichen, wässrigen Träger enthält.

14. Pharmazeutisches Kit, das
- ein gasgefülltes Mikrovesikel oder einen Vorläufer davon enthält, das oder der eine erste Komponente mit Bindungsaffinität für den Fc-Bereich eines Antikörpers enthält, und
- eine zweite Komponente enthält, die einen Fc-Bereich enthält und die über den Fc-Bereich an die erste Komponente binden kann.

15. Pharmazeutisches Kit nach Anspruch 14, das
- einen ersten Behälter, der ein gasgefülltes Mikrovesikel oder einen Vorläufer davon enthält, das oder der eine erste Komponente mit Bindungsaffinität für den Fc-Bereich eines Antikörpers enthält, und
- einen zweiten Behälter enthält, der eine zweite Komponente enthält, die einen Fc-Bereich aufweist und die über den Fc-Bereich an die erste Komponente binden kann.

16. Pharmazeutisches Kit nach Anspruch 15, wobei der erste Behälter einen Vorläufer des gasgefüllten Mikrovesikels in pulverförmiger, trockener Form in Kontakt mit einem Gas enthält.

17. Pharmazeutisches Kit nach Anspruch 15, wobei der zweite Behälter die zweite Komponente in trockener, fester Form oder als eine Suspension in einem physiologisch verträglichen, wässrigen Träger enthält.

18. Pharmazeutisches Kit nach einem der vorangehenden Ansprüche 14 bis 17, das weiterhin einen physiologisch verträglichen, wässrigen Träger enthält.

19. Verfahren zur Herstellung einer Suspension von gasgefüllten Mikrovesikeln wie in Anspruch 12 definiert, bei dem man
- eine Suspension gasgefüllter Mikrovesikel in einem physiologisch verträglichen, wässrigen Träger herstellt, wobei die Mikrovesikel eine erste Komponente mit Bindungsaffinität für einen Fc-Bereich eines Antikörpers enthalten und
- man zu dieser Suspension eine zweite Komponente zumischt, die einen zielführenden Liganden oder ein therapeutisches Mittel enthält und die einen Fc-Bereich eines Antikörpers enthält, der an die erste Komponente binden kann.

## Revendications

1. Microvésicule remplie d'un gaz, comprenant une enveloppe limitante contenant ledit gaz, ladite microvésicule comprenant :
- un premier composant, fixé à ladite enveloppe, ayant une affinité de liaison pour une région Fc d'un anticorps ; et
- un second composant comprenant une région Fc d'un anticorps, lié audit premier composant par ladite région Fc, ledit second composant comprenant un ligand de ciblage ou un agent thérapeutique.

2. Microvésicule remplie d'un gaz selon la revendication 1, dans laquelle le second composant est un anticorps ou une protéine chimérique.

3. Microvésicules remplies d'un gaz selon la revendication 1, dans lesquelles ledit premier composant est une protéine, un anticorps anti-Fc ou un récepteur de Fc.

4. Microvésicules remplies d'un gaz selon la revendication 3, dans lesquelles ladite protéine est choisie dans l'ensemble comprenant une protéine G naturelle ou recombinante, la protéine A et une protéine de fusion A/G recombinante.

5. Microvésicule remplie d'un gaz selon l'une quelconque des revendications précédentes, dans laquelle ledit premier composant est fixé par liaison covalente à ladite enveloppe.

6. Microvésicule remplie d'un gaz selon la revendication 5, dans laquelle le premier composant est lié à un composé amphiphile inclus dans l'enveloppe de la microvésicule.

7. Microvésicule remplie d'un gaz selon la revendication 6, dans laquelle ledit composé amphiphile est un phospholipide, comprenant en option un polymère hydrophile.

8. Microvésicule remplie d'un gaz selon la revendication 7, dans laquelle ledit phospholipide est la phosphatidyléthanolamine.

9. Microvésicule remplie d'un gaz selon l'une quelconque des revendications précédentes, les microvésicules étant des microbulles comprenant un phospholipide dans l'enveloppe limitante.

10. Microvésicule remplie d'un gaz selon l'une quelconque des revendications précédentes, dans laquelle le gaz est choisi parmi l'air, l'azote, l'oxygène, le dioxyde de carbone, l'hydrogène, l'oxyde nitreux, un gaz rare ou inerte, un gaz radioactif, un gaz rare hyperpolarisé, un hydrocarbure de faible masse moléculaire, un éther, une cétone, un ester, un gaz halogéné ou des mélanges de ceux-ci.

11. Microvésicule remplie d'un gaz selon la revendication 10, dans laquelle le gaz est un gaz fluoré, en option en mélange avec de l'azote ou de l'air.

12. Suspension comprenant une pluralité de microvésicules remplies d'un gaz, telles que définies dans l'une quelconque des revendications précédentes, dispersées dans un véhicule aqueux physiologiquement acceptable.

13. Ensemble pharmaceutique comprenant :
- un précurseur d'une microvésicule remplie d'un gaz, comprenant : (i) un premier composant ayant une affinité de liaison pour une région Fc d'un anticorps, et (ii) un second composant, comprenant une région Fc capable de se lier audit premier composant par ladite région Fc, ledit précurseur étant sous la forme d'une composition lyophilisée sèche en contact avec un gaz, et
- un véhicule aqueux physiologiquement acceptable.

14. Ensemble pharmaceutique comprenant :
- une microvésicule remplie d'un gaz, ou un précurseur de celle-ci, comprenant un premier composant ayant une affinité de liaison pour une région Fc d'un anticorps, et
- un second composant, comprenant une région Fc capable de se lier au dit premier composant par ladite région Fc.

15. Ensemble pharmaceutique selon la revendication 14, comprenant :
- un premier contenant, comprenant une microvésicule remplie d'un gaz, ou un précurseur de celle-ci, comprenant un premier composant ayant une affinité de liaison pour la région Fc d'un anticorps ; et
- un second contenant, comprenant un second composant comprenant une région Fc capable de se lier audit premier composant par ladite région Fc.

16. Ensemble pharmaceutique selon la revendication 15, dans lequel ledit premier contenant comprend un précurseur de ladite microvésicule remplie d'un gaz, sous forme sèche pulvérulente, en contact avec un gaz.

17. Ensemble pharmaceutique selon la revendication 15, dans lequel ledit second contenant comprend ledit second composant sous forme de solide sec ou sous forme d'une suspension dans un véhicule aqueux physiologiquement acceptable.

18. Ensemble pharmaceutique selon l'une quelconque des revendications précédentes 14 à 17, comprenant en outre un véhicule aqueux physiologiquement acceptable.

19. Procédé pour la préparation d'une suspension de microvésicules remplies d'un gaz telles que définies dans la revendication 12, qui comprend :
- la préparation d'une suspension de microvésicules remplies d'un gaz dans un véhicule aqueux physiologiquement acceptable, lesdites microvésicules comprenant un premier composant ayant une affinité de liaison pour une région Fc d'un anticorps ; et
- le mélange avec ladite suspension d'un second composant comprenant un ligand de ciblage ou un agent thérapeutique, et comprenant une région Fc d'un anticorps capable de se lier audit premier composant.
